# EUROPEAN PATENT SPECIFICATION

(11) **EP 3 421 519 B1**
(45) Date of publication and mention of the grant of the patent: **21.10.2020**
(21) Application number: 17759282.1
(22) Date of filing: 03.03.2017
(51) Int. Cl.: A61K 9/51, A61K 47/34, A61K 47/42, A61P 35/00, C08G 64/18, C08G 64/30, C08G 63/64, C08G 63/688, A61K 47/55, A61K 47/60, A61K 47/64, A61K 47/69, A61K 47/68, A61K 47/62

(54) **OVARIAN CANCER SPECIFICALLY TARGETED BIODEGRADABLE AMPHIPHILIC POLYMER, POLYMER VESICLE PREPARED THEREBY AND USE THEREOF**
SPEZIELL AUF EIERSTOCKKREBS GERICHTETES, BIOLOGISCH ABBAUBARES AMPHIPHILES POLYMER, DADURCH HERGESTELLTES POLYMERVESIKEL UND VERWENDUNG DAVON
POLYMÈRE AMPHIPHILE BIODÉGRADABLE CIBLANT DE MANIÈRE SPÉCIFIQUE LE CANCER DE L'OVAIRE, VÉSICULE DE POLYMÈRE PRÉPARÉE AVEC CE POLYMÈRE ET UTILISATION ASSOCIÉE

(30) Priority: 04.03.2016 CN 201610123977
(43) Date of publication of application: 02.01.2019
(73) Proprietor: BrightGene Bio-Medical Technology Co., Ltd., Bio-Bay Suzhou Industrial Park Suzhou, Jiangsu 215123 (CN)
(72) Inventor: MENG,Fenghua, Suzhou Jiangsu 215123 (CN); ZOU, Yan, Suzhou Jiangsu 215123 (CN); ZHONG, Zhiyuan, Suzhou Jiangsu 215123 (CN); YUAN, Jiandong, Suzhou, Jiangsu 215123 (CN)
(74) Representative: J A Kemp LLP
(86) International application number: PCT/CN2017/075529
(87) International publication number: WO 2017/148431

(56) References cited:
- EP-A1- 3 392 289
- WO-A1-2015/180655
- WO-A1-2015/180656
- CN-A- 104 031 248
- CN-A- 104 610 538
- CN-A- 104 672 199
- CN-A- 105 669 964

## Description

### TECHNICAL FIELD

The present invention relates to a biodegradable polymer material and application thereof, in particular, to an ovarian cancer specifically targeted biodegradable amphiphilic polymer, a polymeric vesicle prepared thereby and application thereof in the targeted therapy of ovarian cancer,. The present disclosure belongs to the field of medical materials.

### BACKGROUND

Biodegradable polymers have very unique properties and are widely applied in various fields of biomedicine, such as surgical sutures, bone fixation devices, scaffold materials for biological tissue engineering, and carriers for controlled drug release. Synthetic biodegradable polymers mainly include aliphatic polyesters (polyglycolide PGA, polylactide PLA, lactide-glycolide copolymer PLGA, polycaprolactone PCL), polycarbonate (Polytrimethylene carbonate, PTMC), which are the most commonly used biodegradable polymer and have been approved by the US Food and Drug Administration (FDA).

However, the existing biodegradable polymers such as PTMC, PCL, PLA, and PLGA have relatively simple structures and lack modifiable functional groups, as a result it is always difficult to provide a drug carrier for stable circulation. The degradation products of polycarbonate are mainly carbon dioxide and neutral diols, and do not produce acidic degradation products. The functional cyclic carbonate monomer can be copolymerized with cyclic ester monomers such as GA, LA and ε-CL etc., as well as other cyclic carbonate monomers to obtain biodegradable polymers with different properties.

In addition, the biodegradable nano-carrier prepared by the biodegradable polymer of the prior art has the problems of unstable circulation in the body, low uptake for tumor cells, and low drug concentration within cells, which lead to low efficacy of the nanomedicine and toxic side effects. The micelle nanomedicine prepared by functional biodegradable polymers maintains circulation stability in the body, but can only be loaded with hydrophobic small molecular anticancer drugs other than the hydrophilic small molecular anticancer drugs with stronger penetrability, which limits its use as a drug carrier.

Cancer is a main threaten to human health, and its morbidity and mortality are increasing year by year. Ovarian cancer is a malignant tumor of ovarian, which refers to a malignant tumor that grows on the ovarian. 90% to 95% of the ovarian cancer is primary ovarian cancer, and the remaining 5% to 10% of the ovarian cancer is due to the transfer of primary cancer from other parts. Due to the complexity of embryonic development, tissue anatomy and endocrine function for the ovarian, the ovarian tumor can be benign or malignant. Due to the lack of symptoms of specificity in early ovarian cancer and the limited effects of screening, it is very difficult to identify the tissue type of the ovarian tumor and whether it is benign and malignant. Therefore, it is difficult to perform early diagnosis. The tumor confined to the ovarian is found to only account for 30% during laparotomy of ovarian cancer. Most of cases show that the tumor has spread to the bilateral attachments of the uterus, the omentum majus and organs in pelvic cavity. 60% to 70% of the patients are diagnosed as advanced cancers that have poor therapeutic effects. Until now, it is difficult to diagnose and treat ovarian cancer. Therefore, although the incidence of ovarian cancer is lower than that of cervical cancer and endometrial cancer, and the incidence is in the third position of the gynecological malignancies, the mortality rate exceeds the sum of cervical cancer and endometrial cancer, which is the highest in gynecological cancer, thereby the ovarian cancer is a serious threat to the health of women.

In short, ovarian cancer is a female malignant tumor with high incidence. Although the absolute number of cases is not that many, the mortality rate is very high mainly because it is difficult to detect and diagnose at early stage. Most of patients are diagnosed as advanced and thus miss the best time for resection surgery. Further there are many other characteristics, such as low cure rate, easy to transfer and easy to have drug resistance during the therapy of the ovarian cancer. Compared with traditional chemotherapeutic drugs, the distribution in vivo of nanomedicines can be changed, thereby increasing the concentration of drugs in tumors and improving the therapeutic effects. Nanomedicine is a key point and is the hope for the treatment of ovarian cancer. DOXIL (PEGylated liposomal adriamycin) is a liposome vesicle nano drug DOXIL which is approved firstly by FDA and is clinically effective for treating ovarian cancer. However, there are some problems with DOXIL. Firstly the maximum tolerated dose (MTD) is small, so the treatment window is relatively narrow, and it is easy to have toxic side effects. Secondly, DOXIL is based on the passive targeting function of EPR effect, thus it is difficult to transport nanomedicine to all tumor tissues and cells using a common unified mechanism due to the huge individual differences among different tumors. (See: S. Eetezadi, SN. Ekdawi, C. Allen, Adv. Drug Deliv Rev, 2015, 91, 7-22). For different tumors, personalized treatment is particularly important because the surface properties of different tumors are very different, and the same tumors are also very different among different patients; even the tumor cells in the same tumor are different. Therefore, it is necessary to personally design a targeting system suitable for the target tumor, and the drug suitable for one indication cannot be used in other diseases, thus the personalized treatment is particularly important. Therefore, it is necessary to develop an active targeting nano drug against a specific tumor, which has tumor specificity to realize other advantages of the nano drug, for example increasing the effective drug concentration for the tumor cell and improving the therapeutic effect in vitro and in vivo.

WO 2015/180656 discloses a carbonate polymer with disulfur five-membered ring functional group on side chain. However, the polymer is significantly different from the polymer of the present invention in structure. In addition, D1 does not disclose a self-crosslinked polymeric vesicle.

WO 2015/180655 discloses a cyclic carbonate monomer containing disulfur five-membered ring functional group. However, the polymer prepared from the monomer is significantly different from the polymer of the present invention. In addition, D2 does not disclose a self-crosslinked polymeric vesicle.

### SUMMARY

The object of the present invention is to provide an ovarian cancer specifically targeted biodegradable amphiphilic polymer, a polymeric vesicle prepared therefrom, and application thereof as a carrier for an anti-ovarian cancer drug in preparation of ovarian-targeting therapeutic drugs.

The above object is achieved through the following technical solutions:
An ovarian cancer specifically targeted biodegradable amphiphilic polymer, which is prepared by bonding a polymer containing dithiocarbonate monomer with a targeting molecule by Michael addition of amidation reaction; wherein the targeting molecule is GE11 polypeptide or transferrin; and wherein the polymer containing dithiocarbonate monomer has a chemical structure of formula: 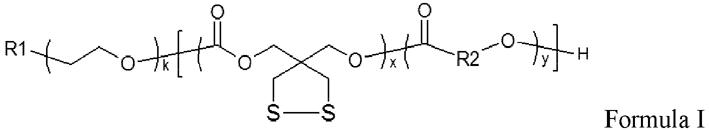 wherein R1 is selected from one of the following groups: 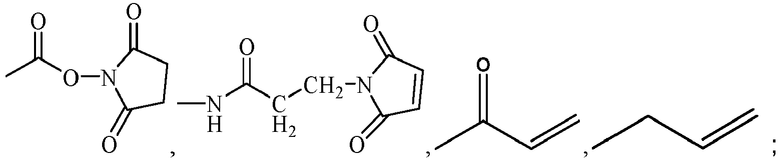 R2 is selected from one of the following groups: 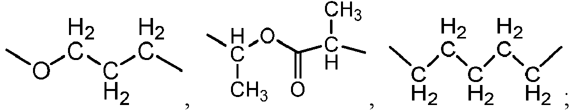 and
wherein k is from 113 to 170, x is from 15 to 45, and y is from 80 to 300, and
the polymer containing dithiocarbonate monomer has a molecular weight of 30 kDa to 55 kDa.

The polymer containing dithiocarbonate monomer disclosed in the present invention has a hydrophobic block that contains a cyclic carbonate unit containing a double-sulfur five-membered ring functional group in the side chain. The polymer containing dithiocarbonate monomer of the present invention can be a diblock polymer as below: 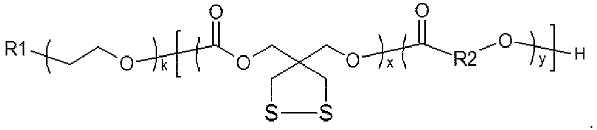 Also disclosed herein is a triblock polymer as below:

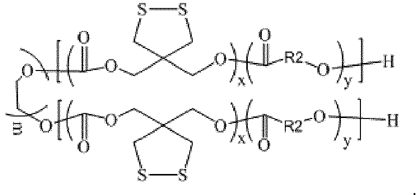

In a preferred embodiment, R1 is selected from one of the following groups:

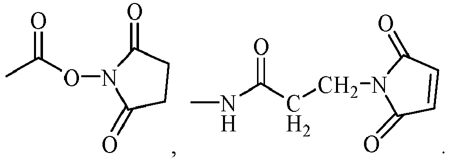

R2 is selected from one of the following groups:

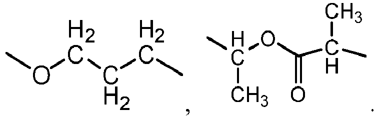

K is 113 to 170, x is 20 to 40, and y is 125 to 250.

The polymer containing dithiocarbonate monomer of the present invention has a molecular weight of 30 kDa to 55 kDa. The polymer of Formula II disclosed herein has a molecular weight of 60 kDa to 95 kDa. The molecular weight of the polymer disclosed in the present invention is controllable, and the constitution and ratio of each structural unit are suitable for self-crosslinked to form a stable polymeric vesicle structure.

The ovarian cancer specifically targeted biodegradable amphiphilic polymer disclosed in the invention has biodegradability, and its hydrophobic portion has a molecular weight which is about three times or more than three times that of the hydrophilic portion. The polymeric vesicle structure can be prepared by a solvent displacement method, a dialysis method or a filming-rehydration method et al. The prepared polymeric vesicle is of nano-size and has a particle size of 50-160 nm, and can be used as a carrier for drugs for treating ovarian cancer. A hydrophobic small molecule anti-ovarian cancer drug such as paclitaxel, docetaxel, adriamycin, olaparib, and gefitinib, etc. can be loaded in a hydrophobic membrane of the vesicle, and a hydrophilic anti-ovarian cancer drug, especially hydrophilic small molecule anticancer drugs such as doxorubicin hydrochloride, epirubicin hydrochloride, or irinotecan hydrochloride or mitoxantrone hydrochloride, can be loaded in a large hydrophilic inner cavity of the vesicle, thereby overcoming the defect that the existing micellar carrier formed from the existing amphiphilic polymer are only applicable for loading hydrophobic drug and the defect that in the prior art there is no carrier which can be efficiently loaded with the hydrophilic small molecular anticancer drugs and be stable *in vivo* circulation.

Also disclosed herein is a polymeric vesicle which is prepared by the above-mentioned polymer containing dithiocarbonate monomer, or prepared by the above-mentioned ovarian cancer specifically targeted biodegradable amphiphilic polymer, or prepared by the above mentioned polymer containing dithiocarbonate monomer and the ovarian cancer specifically targeted biodegradable amphiphilic polymer. For example, by mixing the above-mentioned polymer containing dithiocarbonate monomer with the ovarian cancer specifically targeted biodegradable amphiphilic polymer at different ratios, polymeric vesicles with different targeting densities can be prepared, i.e. obtaining ovarian cancer targeted self-crosslinked polymeric vesicles, thereby increasing the uptake of the polymeric vesicle nanomedicine in ovarian cancer cells. For another example, ovarian cancer targeted polymeric vesicles can also be prepared by coupling a tumor cell specifically targeted molecule to an outer surface of the polymeric vesicle prepared from the polymer containing dithiocarbonate monomer, to increase the uptake in ovarian cancer cells. For example, PEG end of the polymeric vesicle can be bonded to GE11, FA, transferrin or Herceptin, et al. by Michael addition or amidation reaction. Preferably, the polymeric vesicle is prepared by the ovarian cancer specifically targeted biodegradable amphiphilic polymer in an amount of 1 to 40 wt.% by mass and the polymer containing dithiocarbonate monomer.

The polymer containing dithiocarbonate monomer and the ovarian cancer specifically targeted biodegradable amphiphilic polymer of the present invention can be crosslinked with each other without adding any substance, to obtain a self-crosslinked polymeric vesicle. When the polymer is applied as a drug carrier, the most basic and critical property for achieving optimal targeting and therapeutic effects is the long time circulation *in vivo.* The formation of the cross-linked structure is a necessary process for the polymer carrier to circulate in vivo for long time. In the prior art, a stable cross-linked structure of the polymer nano-carrier is formed by adding a crosslinking agent, but the addition of the crosslinking agent can not only increase the complexity for preparing nanomedicines, increase the production cost of nanomedicine, and reduce the final purity of the drug, which is not conducive to the amplification production for clinical application of nanomedicine, but also affect loading efficiency of the drug and drug release level, increase toxic side effects and reduce the biocompatibility of the nano drug loaded in the polymer carrier. The polymer structure firstly disclosed in the present invention can be self-crosslinked without the addition of a crosslinking agent, thereby forming a stable chemical cross-linked structure inside a hydrophobic film in the vesicle. As a result, the polymer can circulate stably *in vivo* for long time. Since no crosslinking agent is added, side effects due to the crosslinking agent are avoided. Furthermore, the drug loaded polymeric vesicles can be decrosslinked quickly in the presence of a large amount of reducing substances within the cell, thereby releasing the maximum amount of drug to kill the ovarian cancer cells after the drug loaded polymeric vesicles reach the tumor and are endocytosed into the cancer cells. Meanwhile, the stability of self-crosslinked polymers equals to or even better than that of the cross-linked polymeric vesicles prepared by crosslinking agent. More importantly, in the present invention, interference of the crosslinking agent to some drugs is avoided. By using the self-crosslinked polymeric vesicles to load drugs, side effects of existing small molecule drugs are avoided, and the application range of the anticancer drug is broadened, and moreover, it can be applied to different individuals with big physiological differences.

The present invention also discloses the ovarian cancer specifically targeted biodegradable amphiphilic polymer for use in a nanomedicine for treating ovarian cancer. Further, the present invention also discloses the above polymeric vesicle for use in a nanomedicine for treating ovarian cancer; in particular the use of the self-crosslinked polymeric vesicle as a carrier in the preparation of a drug for treating ovarian cancer. Due to the use of self-crosslinked polymeric vesicles, it is avoided to use crosslinking agents, which further enhances drug safety and reduces the drug assembly steps. The anti-ovarian cancer nanomedicine prepared based on the polymer of the present invention is a vesicle anti-ovarian cancer nanomedicine.

Based on the implementation of the above technical solution, the present invention has the following advantages compared with the prior art:
1. The biodegradable amphiphilic polymer with a side chain containing double-sulfur disclosed in the invention has biodegradability and excellent targeting property for ovarian cancer, which can be used to prepare polymeric vesicles for loading drugs of different properties, and can be self-crosslinked to form a stable self-crosslinked polymeric vesicle for the nanomedicine without adding any crosslinking agent, thereby overcoming the defects that nanomedicines in prior art are instable during circulation in vivo and easy to release drugs too early to result in toxic side effects.
2. The crosslinking of the self-crosslinked polymeric vesicle for the nanomedicine disclosed by the invention is reversible, i.e. it supports long circulation *in vivo* and can be highly enriched in ovarian cancer cells. However, it can be quickly decrosslinked after entering the ovarian cancer cells and release the drug to kill ovarian cancer cells with high-efficiency and specificity without toxic and side effects, and this overcomes the defects that the crosslinked nanomedicine in the prior art is too stable, and the drug release in the cell is slow, which results in drug resistance.
3. The ovarian cancer specifically targeted biodegradable amphiphilic polymer disclosed in the present invention can be used to prepare self-crosslinked polymeric vesicles without adding any crosslinking agent. The preparation method is simple, thereby overcoming the defects that it is necessary to add substances such as crosslinking agents in preparation of cross-linked nanomedicines in the prior art and the defects that complicated operations and purification processes are required. Therefore this is beneficial to the clinical application of nanomedicines.
4. The self-crosslinked polymeric vesicle prepared by the self-assembly of the ovarian cancer specifically targeted biodegradable amphiphilic polymer disclosed in the invention can be used in the controlled release system of hydrophilic small molecule anticancer drug, thereby overcoming defects that the existing degradable nano-micelle carrier is only suitable for loading hydrophobic small molecule drugs and there is no hydrophilic small molecule anticancer drug in the prior art which can be efficiently loaded and circulates stably. Further, self-crosslinked ploymeric vesicles targeting to the ovarian cancer can be prepared, which have wider application in the efficient and targeted therapy of ovarian cancer.

### BRIEF DESCRIPTION OF THE DRAWINGS

Figure 1 is a graph illustrating the distribution of the particle size (A), the and transmission electron microscopy (B) of the cross-linked polymeric vesicle PEG5k-P(CDC5.8k-co-TMC23k) in the Example 15, the stability test (C) and reduction responsiveness test (D) of the cross-linked polymeric vesicles ;
Figure 2 is a graph illustrating the *in vitro* release of the DOX·HCl -loaded cross-linked polymeric vesicles PEG5k-P(CDC5.8k-co-TMC23k) in Example 20;
Figure 3 is a graph illustrating the in vitro release of the DOX·HCl -loaded cross-linked polymeric vesicles GE11-CLPs in Example 20;
Figure 4 is a graph illustrating the results of toxicity of targeting cross-linked polymeric vesicles GE11-CLPs to SKOV3 ovarian cancer cells in Example 21;
Figure 5 is a graph illustrating the results of toxicity of the DOX·HCl -loaded targeted cross-linked polymeric vesicles GE11-CLPs to SKOV3 ovarian cancer cells in Example 22;
Figure 6 is a graph illustrating the results of semi-lethal toxicity of the DOX·HCl -loaded targeted cross-linked polymeric vesicles GE11-CLPs to SKOV3 ovarian cancer cells in Example 22;
Figure 7 is a graph illustrating the results of endocytosis of SKOV3 ovarian cancer cells by the DOX·HCl -loaded targeted cross-linked polymeric vesicles GE11-CLPs in Example 24;
Figure 8 is a graph illustrating the effects of targeting cross-linked polymeric vesicles GE11-CLPs loaded DOX·HCl to blood circulation in mice in Example 29;
Figure 9 is a graph illustrating biodistribution of the DOX·HCl -loaded targeted cross-linked vesicles GE11-CLPs in mice carrying subcutaneous ovarian cancer in Example 30;
Figure 10 is a graph illustrating the maximum tolerated dose of the DOX·HCl -loaded targeted cross-linked vesicles GE11-CLPs in mice in Example 31;
Figure 11 is a graph illustrating a multi-dose treatment to mice carrying subcutaneous ovarian cancer by the DOX·HCl -loaded targeted cross-linked vesicles GE11-CLPs in Example 32, in which A is a tumor growth curve, B is a tumor image after treatments of the mice. , C shows the change in body weight, and D is the survival curve;
Figure 12 is a graph illustrating a single-dose treatment to mice carrying subcutaneous ovarian cancer by the DOX·HCl -loaded targeted cross-linked vesicles GE11-CLPs in Example 33, in which the A is a tumor growth curve, B is a tumor image after treatment in mice. , C is the weight change curve, and D is the survival curve.

### DETAILED DESCRIPTION

The present invention will be further described below in combination with the examples and the accompanying drawings:

### Example 1 Synthesis of a cyclic carbonate monomer (CDC) which contains a double-sulfur five-membered ring function group

Sodium hydrosulfide monohydrate (28.25 g, 381.7 mmol) was dissolved in 400 mL of N,N-dimethyl formamide (DMF) and heated to 50°C to achieve complete dissolution. Then dibromo neopentyl glycol (20 g, 76.4 mmol) was added dropwise to carry out reaction which lasted for 48 hours. The resultant was distilled under a reduced pressure to remove the solvent DMF, then diluted with 200 mL of distilled water, and extracted four times with 250 mL of ethyl acetate each time to obtain an organic phase. The organic phase was rotary evaporated to give a yellow viscous compound A, with a yield of 70%. The compound A was dissolved in 400 mL of tetrahydrofuran (THF) and was allowed to stand in air for 24 hours, as a result intermolecular thiol was oxidized to a sulfur-sulfur bond to give a compound B with a yield >98%. The compound B (11.7 g, 70.5 mmol) was dissolved in dried THF (150 mL) and stirred until completely dissolved under nitrogen. It was then cooled to 0°C. Ethyl chloroformate (15.65 mL, 119.8 mmol) was added and then Et₃N (22.83 mL, 120.0 mmol) was added dropwise. After the addition was completed, the system was further reacted for 4 h under ice-water bath conditions. After the reaction was completed, Et₃N·HCl was filtered off, and the filtrate was concentrated by rotary evaporation, then it was recrystallized for several times by using diethyl ether to obtain a yellow crystalline which is a cyclic carbonate monomer (CDC) that contains a double-sulfur five-membered ring functional group, with a yield of 64 %.

### Reference Example 2 Synthesis of a diblock polymer PEG5k-P(CDC5.8k-co-TMC23k)

Under nitrogen atmosphere, 0.1 g (0.52 mmol) of CDC monomer and 0.4 g (4.90 mmol) of trimethylene carbonate (TMC) were dissolved in 5 mL of dichloromethane and placed to a seal reactor, and then 0.12 g (0.02 mmol) CH₃O-PEG5000 and 0.5 mL of catalyst solution of bi(bistrimethylsilyl)amine zinc in dichloromethane (0.1 mol/L) were added, then the reactor was sealed and transferred out of the glove box. After 2 days of reaction in oil bath at 40°C, the reaction was quenched with glacial acetic acid, precipitated in iced diethyl ether, and finally filtered and vacuum dried to give PEG5k-P(CDC 5.8k-co-TMC23k). ¹H NMR (400 MHz, CDCl₃): 2.08 (t, -COCH₂CH₂CH₂O-), 3.08(s, -CCH₂), 3.30(m, -OCH₃), 3.65(-OCH₂CH₂O-), 4.28(t, -COCH₂CH₂CH₂O-), 4.31 (m, -CCH₂). The nuclear magnetic resonance was used to calculate the following: k = 114, x = 30.2 and y = 225.5. Molecular weight measured by GPC: 45.6 kDa, molecular weight distribution: 1.53.

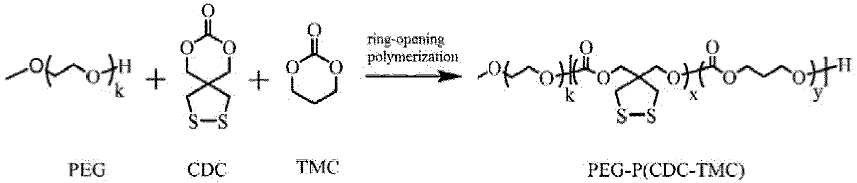

### Example 3 Synthesis of a diblock polymer Mal-PEG6k-P(CDC4.8k-co-TMC19.2k)

Under nitrogen atmosphere, 0.1 g (0.52 mmol) of CDC monomer and 0.4 g (3.85 mmol) of TMC were dissolved in 3 mL of dichloromethane and placed to a seal reactor, and then 0.12 g (0.02 mmol) of Mal-PEG6000 and 0.1 mol/L catalyst solution of bi(bistrimethylsilyl)amine zinc in dichloromethane (0.1 mol/L) were added, then the reactor was sealed and transferred out of the glove box. After 2 days of reaction in oil bath at 40°C , the reaction was quenched with glacial acetic acid, precipitated in iced diethyl ether, and finally filtered and vacuum dried to give Mal-PEG6kP(CDC4.8k-co-TMC l 9.2k). ¹H NMR (400 MHz, CDCl₃): 2.08 (t, -COCH₂CH₂CH₂O-), 3.08 (s, -CCH₂), 3.30 (m, -OCH₃), 3.65 (t, -OCH₂ CH₂O-), 4.28 (t, -COCH₂CH₂CH₂O -), 4.31 (m, -CCH₂), and 6.70 (s, Mal). The nuclear magnetic resonance was used to calculate the following: k = 136, x = 25, and y = 188. Molecular weight measured by GPC: 38.6 kDa, molecular weight distribution: 1.42.

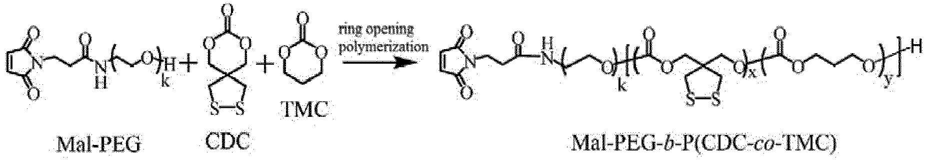

### Example 4 Synthesis of a diblock polymer NHS-PEG6.5k-P(CDC6k-co-TMC22.6k)

Under nitrogen atmosphere, 0.1 g (0.52 mmol) of CDC monomer and 0.42 g (4.12 mmol) of TMC were dissolved in 5 mL of dichloromethane and palcaed to a seal reactor, and then 0.11 g (0.017 mmol) of NHS-PEG6500 and 0.5 mL of catalyst solution of bi(bistrimethylsilyl)amine zinc in dichloromethane (0.1 mol/L) were added, then the reactor was sealed and transferred out of the glove box. After 2 days of reaction in oil bath at 40°C , the reaction was quenched with glacial acetic acid, precipitated in iced diethyl ether, and finally filtered and vacuum dried to give NHS-PEG 6.5k-P(CDC6k-co-TMC22.6k). ¹H NMR (400 MHz, CDCl₃): 2.08 (t, -COCH₂CH₂CH₂O-), 3.08 (s, -CCH₂), 3.30 (m, -OCH₃), 3.65 (-OCH₂ CH₂O-), 4.28 (t, -COCH₂CH₂CH₂O-), 4.31 (m, -CCH₂), and 2.3 (s, NHS). The nuclear magnetic resonance was used to calculate the following: k = 148, x = 31.3, and y = 221.6. Molecular weight measured by GPC: 51.3 kDa, molecular weight distribution: 1.43.

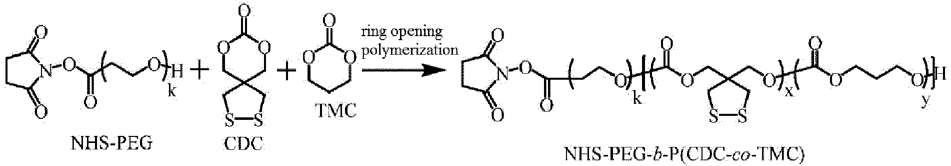

### Reference Example 5 Synthesis of a diblock polymer PEG7.5k-P(CDC5.8k-co-TMC20.0k)

Under nitrogen atmosphere, 60 mg (0.31 mmol) of CDC monomer and 0.2 g (1.93 mmol) of TMC were dissolved in 1 mL of dichloromethane and placed to a seal reactor, and then 75 mg (0.01 mmol) of CH₃O-PEG7500 and 0.5 mL of catalyst solution of bi(bistrimethylsilyl)amine zinc in dichloromethane (0.1 mol/L) were added, then reacted in oil bath at 40°C for 2 days. Subsequent processing steps were the same as those in Example 2, to obtain PEG7.5k-P(CDC5.8k-co-TMC20.0k). The reaction formula and the characteristic peaks of ¹H NMR are the same as Example 2. The nuclear magnetic resonance was used to calculate the following: k=170, x=30, and y=196. Molecular weight measured by GPC: 54.5 kDa, molecular weight distribution: 1.36.

### Reference Example 6 Synthesis of a diblock polymer PEG5k-P(CDC3.9k-co-LA18.0k)

Under nitrogen atmosphere, 0.08 g (0.42 mmol) of CDC and 0.3 g (2.1 mmol) of lactide (LA) were dissolved in 2 mL of dichloromethane and placed to a seal reactor, and then 0.1 g (0.02 mmol) of CH₃O-PEG5000 and 0.1 mol/L of catalyst bi(bistrimethylsilyl)amine zinc in dichloromethane (0.1 mL) were added, then reacted in oil bath at 40°C for 2 days, and subsequent processing steps were the same as those in Example 2 to obtain PEG5k-P(CDC3.9k-co-LA14.6k). ¹H NMR (400 MHz, CDCl₃): 1.59 (s, -COCH(CH₃)O-), 3.08 (s, -CCH₂), 3.30 (m, -OCH₃), 3.65 (-OCH₂ CH₂O-), 4.31 (m, -CCH₂), 5.07 (s, -COCH(CH₃)O-). The nuclear magnetic resonance was used to calculate the following: k=114, x=20, and y=125. Molecular weight measured by GPC: 34.3 kDa, molecular weight distribution: 1.32.

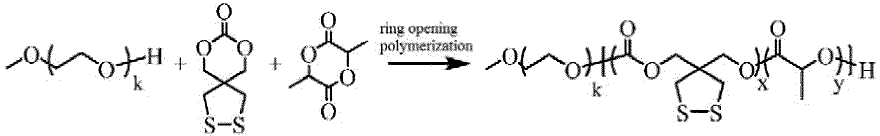

### Reference Example 7 Synthesis of a diblock polymer PEG6.5k-P(CDC5.8k-co-LA28.3k)

Under nitrogen atmosphere, 0.1 g (0.57 mmol) of CDC and 0.5 g (3.5 mmol) of LA were dissolved in 3 mL of dichloromethane and placed to a seal reactor, and then 0.11 g (0.015 mmol) of CH₃O-PEG6500 and 0.5 mL of catalyst solution of bi(bistrimethylsilyl)amine zinc in dichloromethane (0.1 mol/L) were added, then reacted in oil bath at 40°C for 2 days, and subsequent processing steps were the same as those in Example 6 to obtain PEG6.5k-P(CDC5.8k- co-LA28.3k). The nuclear magnetic resonance was used to calculate the following: k=148, x=30, and y=190. Molecular weight measured by GPC: 42.4 kDa, molecular weight distribution: 1.43.

### Example 8 Synthesis of a diblock polymer Mal-PEG6k-P(CDC3.6k-co-LA18.6k)

Under nitrogen atmosphere, 0.1 g (0.52 mmol) of CDC and 0.5 g (5.56 mmol) of LA were dissolved in 4 mL of dichloromethane, and placed to a seal reactor, and then 0.15 g (0.025 mmol) of Mal-PEG6000 and 0.1 mol/L of catalyst solution of bi(bistrimethylsilyl)amine zinc in dichloromethane (0.1 mL) were added, then reacted in oil bath at 40°C for 2 days, and subsequent processing steps were the same as those in Example 2 to obtain Mal-PEG6k-P(CDC3.6k- co-LA18.6k). ¹H NMR (400 MHz, CDCl₃): 1.59 (s, -COCH(CH₃) O-), 3.08 (s, -CCH₂), 3.30 (m, -OCH₃), 3.65 (t, -OCH₂ CH₂O-), 4.31 (m, -CCH₂), 5.07 (s, -COCH(CH₃) O-), and 6.70 (s, Mal). The nuclear magnetic resonance was used to calculate the following: k = 136, x = 20, and y = 129. Molecular weight measured by GPC: 32.5 kDa, molecular weight distribution: 1.44.

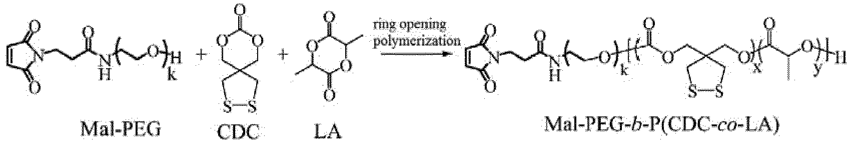

### Reference Example 9 Synthesis of

### triblock polymer P (CDC3.8k-co-TMC18.8k)-PEG10k-P(CDC3.78k-co-TMC18.8k)

Under nitrogen atmosphere,0.8 g (7.84 mmol) of TMC and 0.16 g (0.83 mmol) of CDC were dissolved in 8 mL of dichloromethane and placed to a seal reactor, and then 0.2 g (0.04 mmol) of HO-PEG-OH10000 and 1 mL of catalyst solution of bi(bistrimethylsilyl)amine zinc in dichloromethane (0.2 mol/L), were added, then reacted in oil bath at 40°C for 2 days, and subsequent processing steps were the same as those in Example 2, to obtain a triblock polymer P(CDC3.8k-co-TMC18.8k)-PEG10k-P(CDC3.8k-co-TMC18.8k). The ¹H NMR characteristic peak was the same as Example 2. The nuclear magnetic resonance was used to calculate the following: m = 227, x = 20, and y = 184. Molecular weight measured by GPC: 92.3 kDa, molecular weight distribution: 1.46.

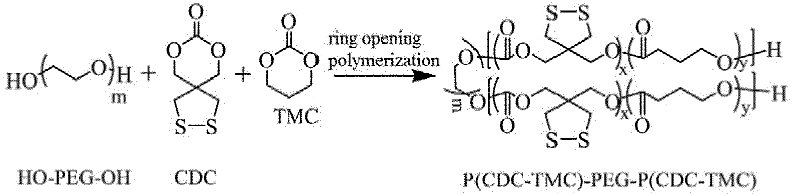

### Example 10 Synthesis of a diblock polymer NHS-PEG7.5k-P(CDC3.8k-co-LA13.8k)

Under nitrogen atmosphere, 0.1 g (0.52 mmol) of CDC and 0.4 g (2.8 mmol) of LA were dissolved in 3 mL of dichloromethane, and placed to a seal reactor, and then 0.013 mmol of NHS-PEG 7500 and 1 mL of catalyst solution of (bis-trimethylsilyl)amine zinc in dichloromethane (0.1 mol/L) were added. Then the reactor was sealed and transferred out of the glove box. The mixture reacted in oil bath at 40°C for 2 days. Subsequent processing steps were the same as those in Example 2 to obtain NHS-PEG7.5k-P(CDC4.8k-co-LA19.0k). ¹H NMR (400 MHz, CDCl₃): 1.59 (s, -COCH(CH₃) O-), 3.08 (s, -CCH₂), 3.30 (m, -OCH₃), 3.65 (t, -OCH₂ CH₂O-) , 4.31 (m, -CCH₂), 5.07 (s, -COCH(CH₃)O-) and 2.3 (s, NHS). The nuclear magnetic resonance was used to calculate the following: k = 170, x = 20, and y = 96. Molecular weight measured by GPC: 42.3 kDa, molecular weight distribution: 1.45.

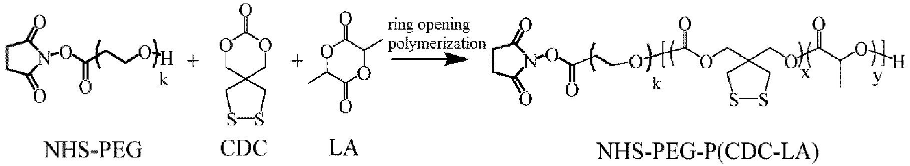

A variety of polymer containing dithiocarbonate monomers can be prepared by the similar preparation methods described above. The proportion and characterization of the raw materials are shown in Table 1.

**Table 1 Preparation condition for each polymer, and nuclear magnetic resonance and GPC characterization results of the product**

| Polymer | amount for preparation (mmol) | | | repeated unit(nuclear magantic) | | | Molecular weight (kg/mol) | | PDI |
|---|---|---|---|---|---|---|---|---|---|
| | PEG hydro xyl | CDC | TMC ,LA, CL | K or m | x | y | Nucl ear magn atic | GPC | GPC |
| PEG5k-P(CDC4.9k-co-TMC19k) | 0.02 | 0.52 | 3.85 | 114 | 26 | 186 | 28.9 | 34.5 | 1.48 |
| Mal-PEG6k-P(CDC4.8 k-co-TMC19.2k) | 0.017 | 0.52 | 3.85 | 136 | 25 | 188 | 29.9 | 38.6 | 1.42 |
| NHS-PEG6.5k-P(CDC4.6k-co-TMC18.6k) | 0.015 | 0.52 | 3.85 | 145 | 24 | 182 | 29.7 | 37.6 | 1.38 |
| PEG5k-P(CDC5.8k-co-TMC23k) | 0.02 | 0.52 | 4.90 | 114 | 30 | 226 | 33.8 | 45.6 | 1.53 |
| NHS-PEG6.5k-P(CDC6 k-co-TMC22.6k) | 0.017 | 0.52 | 4.12 | 148 | 31 | 222 | 35.1 | 51.3 | 1.43 |
| PEG7.5k-P(CDC5.8k-c o-TMC20.0k) | 0.01 | 0.31 | 1.93 | 170 | 30 | 125 | 33.3 | 54.5 | 1.36 |
| PEG6. 5k-P(CDC5.8k-c o-LA28.3k) | 0.015 | 0.57 | 3.47 | 148 | 30 | 200 | 36.6 | 42.4 | 1.43 |
| PEG5k-P(CDC3.7k-co-LA19.8k) | 0.02 | 0.42 | 2.08 | 114 | 20 | 125 | 23.3 | 24.3 | 1.32 |
| Mal-PEG6k-P(CDC3.6 k-co-LA 18.6k) | 0.025 | 0.52 | 5.56 | 136 | 20 | 129 | 28.6 | 32.5 | 1.44 |
| P(CDC3.8k-co-TMC18. 8k)-PEG12k-P(CDC3.8 k-co-TMC18.8k)* | 0.01 | 0.21 | 1.96 | 272 | 20 | 184 | 57.2 | 84.5 | 1.53 |
| P(CDC3.8k-co-LA18.8 k)-PEG10k-P(CDC3.8k -co-LA18.8k)* | 0.05 | 0.52 | 2.08 | 227 | 20 | 131 | 55.2 | 92.3 | 1.46 |
| NHS-PEG7.5k-P(CDC3 .8k-co-LA18.8k) | 0.013 | 0.52 | 2.80 | 170 | 20 | 131 | 24.1 | 42.3 | 1.45 |
| P(CDC3.8k-co-TMC18. 8k)-PEG15k-P(CDC3.8 k-co-TMC18.8k)* | 0.04 | 0.83 | 7.84 | 340 | 20 | 184 | 70.2 | 121. 9 | 1.61 |
| AA-PEG5k-P(CDC3.8k -co-TMC 19.3k) | 0.02 | 0.42 | 3.92 | 114 | 20 | 189 | 28.1 | 34.6 | 1.43 |
| PEG5k-P(CDC5.8k-co-TMC18.7k) | 0.02 | 0.52 | 3.92 | 114 | 30 | 183 | 29.5 | 36.8 | 1.51 |
| PEG5k-P(CDC5.7k-co-LA18.8k) | 0.02 | 0.52 | 2.08 | 114 | 30 | 131 | 29.5 | 40.9 | 1.42 |

### Starred examples are included for reference purposes.

### Example 11 Synthesis of a targeted polymer transferrin-PEG7.5k-P(CDC3.8k-co-LA13.8k)

Transferrin-conjugated polymer is synthesized by two steps. A first step is to prepare Mal-PEG7.5k-P(CDC3.8k-co-LA13.8k) as described in Example 8; and a second step is to bond it with transferrin via Michael reaction. The above described polymer Mal-PEG7.5k-P(CDC3.8k-co-LA13.8k) was firstly dissolved in DMF, into which 2 times molar amount of transferrin was added to carry out reaction at 30°C for two days, and then the reaction products were dialyzed, and lyophilized to obtain transferrin-PEG6.5k-P(CDC3.8k-co-LA13.8k). The transferrin grafting rate was calculated as 95% by the nuclear magnetic resonance and BCA protein kit test.

### Reference Example 12 Synthesis of a targeted polymer Herceptin-PEG6k-P(CDC3.6k-co-LA18.6k)

A human epidermal growth factor antibody Herceptin-conjugated polymer is synthesized by three steps. The first step is to prepare Mal-PEG6k-P(CDC3.6k-co-LA18.6k) as in the Example 8. the second step is to convert the terminal group to an amino group by reacting Mal-PEG6k- P (CDC3.6k-co-LA18.6k) with cysteamine through Michael addition reaction; and the third step is to bond a carboxyl of Herceptin to the amino group by amidation reaction. In detail, the polymer obtained in the previous step is firstly dissolved in 0.5 ml of DMF, 2 ml of boric acid buffer solution (pH 8.0) was added and then 2 times molar amount of Herceptin was added to carry out reaction at 30°C for two days, and then the reaction products were dialyzed and lyophilized to obtain the Herceptin-PEG6k-P(CDC3.6k-co-LA18.6k). The transferrin grafting rate was calculated as 96% by the nuclear magnetic resonance and BCA protein kit test.

### Reference Example 13 Synthesis of a targeted Polymer FA-PEG6.5k-P(CDC6k-co-TMC22.6k)

Folic acid (FA) coupled polymer is synthesized by two steps. The first step is to prepare NHS-PEG6.5k-P(CDC6k-co-TMC22.6k) as in the Example 4. The second step is to bond the amino group of FA to NHS-PEG6.5k-P(CDC6k-co-TMC22.6k) by amidation reaction. In detail, the above mentioned polymer is firstly dissolved in DMF, into which 2 times molar amount of FA was added to carry out reaction at 30°C for two days, and then the reaction products were dialyzed to remove free FA and lyophilized to obtain FA-PEG6.5k-P(CDC6k-co-TMC22.6k). The transferrin grafting rate was calculated as 88% by the nuclear magnetic resonance.

### Example 14 Synthesis of a targeted Polymer GE11-PEG6.5k-P(CDC6k-co-TMC22.6k)

Cyclic polypeptide YHWYGYTPQNVI (GE11) coupled polymer is prepared by two steps. The first step is to prepare NHS-PEG6.5k-P(CDC6k-co-TMC22.6k) as in the Example 4. The second step is to bond amino group of GE11 to the above product by amidation reaction. In detail, the above mentioned polymer is firstly dissolved in DMF, into which 2 times molar amount of GE11 was added to carry out reaction at 30°C for two days, and then the reaction products were dialyzed to remove free GE11, and lyophilized to obtain GE11-PEG6.5k-P(CDC6k-co-TMC22.6k). The GE11 grafting rate was calculated as 96% by the nuclear magnetic resonance and BCA protein kit test.

According to the methods of Examples 11 to 13, the polymer containing dithiocarbonate monomer in Examples 2 to 10 and Table 1 is bonded with a targeting molecule to prepare an ovarian cancer specifically targeted degradable amphiphilic polymer.

### Example 15 Preparation of self-crosslinked polymeric vesicles

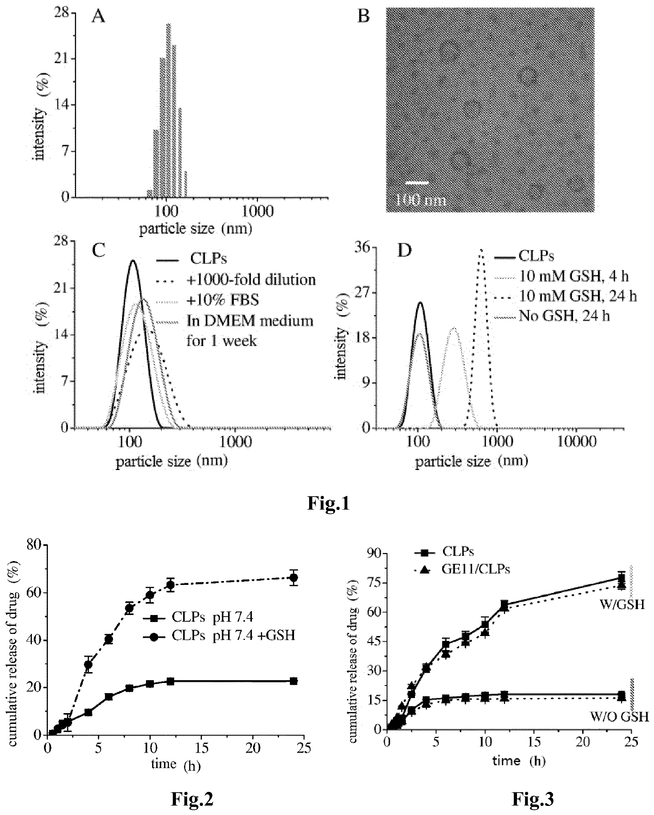

Polymeric vesicles were prepared by solvent displacement. 100 µL of PEG5k-P(CDC5.8k-co-TMC23k) in DMF solution (10 mg/mL) was added dropwise to 900 µL of phosphate buffer solution (PB, 10 mM, pH 7.4), the mixed solutions were placed in a shaker at 37°C (200 rpm) overnight for self-crosslinked, and then dialyzed overnight in a dialysis bag (MWCO 7000) with five times replacement of media PB. Figure 1 is a graph illustrating the particle size distribution (A) and electron transmission microscopy (B) of the self-crosslinked polymeric vesicle PEG5k-P(CDC5.8k-co-TMC23k), a stability test (C) and a reduction response test (D) of the cross-linked vesicles. The size of the self-crosslinked polymeric vesicles obtained was determined by dynamic light scattering particle size analyzer (DLS), and the formed nano-vesicles have a particle size of 130 nm, and a very narrow particle size distribution, as shown in Fig. 1A. It can be found from Fig. B that, TEM shows that the nanoparticle has a hollow vesicle structure, and the particle size and distribution remains unchanged when the self-crosslinked polymeric vesicles were highly diluted in the presence of fetal bovine serum (Fig. 1C). However, in the case of simulation of tumor cell reduction environment, the self-crosslinked polymeric vesicles released and de-crosslinked rapidly (Figure ID). Thus, the obtained vesicles can be self-crosslinked and have a property of reduction-sensitive decrosslinking. Using the same preparation method, PEG5k-P(CDC4.9k-co-TMC19k) can form self-crosslinked nanovesicles with a particle size of 100 nm and a particle size distribution of 0.1.

### Example 16 Preparation of self-crosslinked polymeric vesicles by dialysis and thin-film hydration methods

Self-crosslinked polymeric vesicles were prepared by dialysis method. 100 µL of PEG5k-P(CDC5.8k-co-TMC23k) in DMF (10 mg/mL) was placed in a dialysis bag (MWCO 7000) and placed in a shaker at 37°C(200 rpm) overnight for crosslinking in presence of PB, and then dialyzed for 24 hours in PB for five times replacement of media PB. The cross-linked vesicles have a particle size around 60 nm and a particle size distribution of 0.08 measured by DLS.

Self-crosslinked polymeric vesicles were prepared by film hydration method. 2 mg of PEG5k-P(CDC5.8k-co-TMC23k) was dissolved in 0.5 mL of organic solvent with low boiling point, such as dichloromethane or acetonitrile, in a 25 ml sharp-bottomed flask, and rotary evaporated to form a film at the bottom. Then the flask was evacuated for 24 hours under a vacuum of 0.1 mBar. 2 mL of PB (10 mM, pH 7.4) was added and the solution was stirred, then a film was peeled off and pulverized at 37°C, and then sonicated for 20 minutes (200 rpm), and continuously stirred for 24 hours. The obtained vesicles were self-crosslinked. The size of the self-crosslinked polymeric vesicles measured by DLS was about 160 nm and the particle size distribution was 0.24.

### Example 17 Preparation of self-crosslinked polymeric vesicles GE11-CLPs based on PEG5k-P(CDC5.8k-co-TMC23k) and GE11-PEG6.5k-P(CDC6k-co-TMC22.6k)

The targeting polymer GE11-PEG6.5k-P(CDC6k-co-TMC22.6k) obtained in Example 14 and the PEG5k-P(CDC5.8k-co-TMC23k) obtained in Example 2 were dissolved in DMF. The self-crosslinked polymeric vesicle was prepared by a solvent displacement method as in Example 15. The PEG molecular weight of the targeting polymer is larger than the non-targeting PEG, which ensures that the targeting molecule has a better expenditure surface. The two kinds of polymers can be mixed in different ratios to prepare self-crosslinked polymeric vesicles GE11-CLPs with different targeting molecular content on the surface. The targeting polymer GE11-PEG6.5k-P(CDC6k-co-TMC22.6k) was used in an amount of 10 wt.% to 30 wt.%. The self-crosslinked polymeric vesicles have a particle size of about 85-130 nm and a particle size distribution of 0.01-0.20 determined by DLS.

### Reference Example 18 Preparation of self-crosslinked polymeric vesicles FA-CLPs based on FA-PEG6.5k-P(CDC6k-co-TMC22.6k) and PEG5k-P(CDC5.8k-co-TMC23k)

A solution of 1.6 mg of the PEG5k-P(CDC5.8k-co-TMC23k) prepared in Example 2 dissolved in DMF (10 mg/mL) and 0.4 mg of FA-PEG6.5k-P(CDC6k- co-TMC22.6k) obtained in Reference Example 13 dissolved in 0.5 mL of an organic solvent with low boiling point, such as dichloromethane or acetonitrile were used to prepare the self-crosslinked polymeric vesicle through the film hydration method as in Example 17. The self-crosslinked polymeric vesicles have a particle size of about 88 nm and a particle size distribution of 0.08. The two kinds of polymers can be mixed in different ratios to prepare self-crosslinked polymeric vesicles FA-CLPs with different targeted molecular content on the surface. The FA-PEG6.5k-P(CDC6k-co-TMC22.6k) was used in an amount of 5-30 wt.%. The self-crosslinked polymeric vesicles have a particle size of about 85-130 nm and a particle size distribution of 0.01-0.20.

### Example 19 Preparation of self-crosslinked polymeric vesicles transferrin-CLPs with surface coupled to transferrin

Mal-PEG6k-P(CDC3.6k-LA18.6k) prepared in Example 8 was mixed with P(CDC3.8k-LA18.8k)-PEG4k-P(CDC3.8k-LA18.8k), then 0.5 ml of 4M boric acid buffer solution (pH 8.0) was added to adjust the pH of the solution to 7.5-8.0, and transferrin was added in an amount of 1.5 times the molar amount of Mal. They bonded to each other through Michael addition reaction, and reacted at 30°C for two days, and then the reaction products were dialyzed, and prepared into vesicles transferrin-CLPs according to the dialysis method in Example 16. The vesicles were measured by DLS to have a particle size of 115 nm and a particle size distribution of 0.12. The graft ratio of the polypeptides was calculated via nuclear magnetic and BCA protein kits to be 94%. The targeting polymer and non-targeting polymer were fed by mass ratio in order to prepare self-crosslinked polymeric vesicles transferrin-CLPs from different targeting molecules, wherein the vesicles transferrin-CLPs have a particle size of about 85-130 nm, and a particle size distribution of 0.01-0.20.

A variety of self-crosslinked polymeric vesicles can be prepared by similar preparation methods described above, wherein the proportions and characterization of the raw materials are shown in Table 2.

**Table 2 Preparation and characterization of self-crosslinked polymeric vesicles**

| Polymer | solvent displacem ent method | dialys is metho d | filming-rehydr ation method | siz e (n m) | particle size distribut ion |
|---|---|---|---|---|---|
| PEG5k-P(CDC3.9k-*co*-LA18.6k) | √ | | | 130 | 0.14 |
| PEG5k-P(CDC5.8k-co-TMC23k) | √ | | | 102 | 0.11 |
| FA/PEG5k-P(CDC5.8k-co-TMC23k)* | | | √ | 88 | 0.08 |
| GE11/PEG5k-P(CDC5.8k-co-TMC23k) | | √ | | 96 | 0.18 |
| PEG5k-P(CDC4.9k-TMC14.1k) | | | √ | 67 | 0.12 |
| PEG6.5k-P(CDC5.8k-LA28.3k) | √ | | | 158 | 0.20 |
| PEG7.5k-P(CDC3.9k-TMC14.1k) | | √ | | 53 | 0.15 |
| P(CDC3.9k-TMC18.8k)-PEG5k-P(CDC3.9k-TMC18.8k)* | | | √ | 143 | 0.21 |
| N₃-PEG7k-P(CDC4.7k-PDSC12.6k) | | √ | | 100 | 0.13 |
| AA-PEG5k-P(CDC3.9k-PDSC14.8k) | √ | | | 152 | 0.18 |
| Ally-PEG6k-P(CDC3.9k-CL14.2k) | | | √ | 124 | 0.21 |

### Starred examples are included for reference purposes.

### Example 20 Drug loading and in vitro release of self-crosslinked polymeric vesicles

PEG5k-P(CDC5.8k-co-TMC23k) self-crosslinked polymeric vesicles were prepared by solvent displacement method. DOX·HCl was loaded by pH gradient method utilizing different pH inside and outside the vesicles to have the hydrophilic DOX·HCl encapsulated. 100 µL of PEG5k-P(CDC5.8k-co-TMC23k) in DMF solution (10 mg/mL) was added dropwise to 900 µL sodium citrate/citrate acid buffer solution (10 mM, pH 4.0), and placed in a shaker at 37°C (200 rmp) for 5 hours, then 0.05 mL of PB (4 M, pH 8.1) was added to establish a pH gradient, then the DOX·HCl was immediately added and placed in the shaker for 5-10 hours to allow the drug to enter the vesicles while the vesicles were self-crosslinked. Finally, they were dialyzed overnight in a dialysis bag (MWCO 7000), and the PB (10 mM, pH 7.4) for dialysis medium was changed five times. The self-crosslinked polymeric vesicles carrying different proportions of the drug (10%-30%) have a particle size of 108-128 nm and a particle size distribution of 0.10-0.14. Encapsulation efficiency of DOX·HCl measured by Fluorescence spectrometer was 68% to 85%. The in vitro release experiments of DOX·HCl was performed by shaking (200 rpm) at a constant temperature shaker of 37°C with three parallel samples in each group. In the first group, DOX·HCl-loaded self-crosslinked polymeric vesicles in PB (10 mM, pH 7.4) added with 10 mM GSH simulating intracellular reducing environment; the second group, DOX·HCl-loaded self-crosslinked polymeric vesicles in PB (10 mM, pH 7.4). The concentration of drug-carrying self-crosslinked polymeric vesicles was 30 mg/L, wherein 0.6 mL was placed in a dialysis bag (MWCO: 12,000), and each tube was filled with 25 mL of the corresponding dialysis solvent. 5.0 mL dialysis solvent outside the dialysis bag was taken out at the scheduled time interval for test and 5.0 mL of the corresponding media was supplemented to the tube. The concentration of the drug in the solution was measured by a fluorometer. Figure 2 shows the relationship between the cumulative release of DOX·HCl in respective of time. It can be found from the figure that the release of DOX·HCl in the sample added with GSH is significantly faster than that in the samples without GSH, indicating that the drug-carrying self-crosslinked polymeric vesicles are capable of releasing drug effectively in the presence of 10 mM GSH.

The DOX·HCl-loaded self-crosslinked polymeric vesicle PEG5k-P(CDC4.9k-co-TMC19k) was prepared by the same method as above. The self-crosslinked polymeric vesicles comprising different proportions of the drug (10% to 30%) have a particle size of 100-125 nm and a particle size distribution of 0.10-0.14. Encapsulation efficiency of DOX·HCl measured by Fluorescence spectrometer was 60%-80%.

Self-crosslinked polymeric vesicles Ally-PEG6k-P(CDC2.9k-CL14.2k) were prepared by solvent displacement. 10 µL of paclitaxel PTX in DMF (10 mg/mL) and 90 µL of Ally-PEG6k-P(CDC2.9k-CL14.2k) in DMF (10 mg/mL) were mixed, then added dropwise to 900 µL of phosphate buffer (10 mM, pH 7.4, PB), and placed in a shaker at 37°C (200 rpm) for self-crosslinked overnight, then placed in a dialysis bag (MWCO 7000) for dialysis overnight, during which water was refreshed for five times. The dialysis medium is PB (10 mM, pH 7.4). The PTX is in an amount of 0-20 wt.%, and the obtained self-crosslinked polymeric vesicles have a particle size of 130-170 nm and a particle size distribution of 0.1-0.2. The vesicle structure measured by TEM has decrosslinking properties sensitive to reduction. The encapsulation efficiency of PTX is 50%-70%. The in vitro release experiment was designed as above, and the release of hydrophobic drugs in the sample with GSH was significantly faster than that in the sample without GSH.

Drug-loaded self-crosslinked polymeric vesicles FA-CLPs based on PEG6.5k-P(CDC6k-co-TMC22.6k) were prepared by filming-rehydration method, and DOX·HCl was loaded by pH gradient method. 1.6 mg of PEG5k-P(CDC5.8k-co-TMC23k) and 0.4 mg of FA-PEG6.5k-P(CDC6k-co-TMC22.6k) were dissolved in 0.5 mL of low boiling organic solvent such as dichloromethane or acetonitrile, and rotary evaporated in a 25 ml sharp-bottomed flask to form a film at the bottom. Then the flask was evacuated under a vacuum of 0.1 mBar for 24 hours. 2 mL of sodium citrate/citrate acid buffer solution (10 mM, pH 4.0) was added, then the film was peeled off the bottom at 37 °C and stirred to pieces. Ultrasonic irradiation (200 rpm) was carried out for 20 minutes, stirring for 24 hours, then the vesicles were self-crosslinked. The crosslinking vesicles were measured by DLS to have a particle size of 90 nm and a particle size distribution of 0.10. 0.05 mL of PB (4M, pH 8.1) was added to the above solution of vesicles to establish a pH gradient, followed by immediate addition of DOX·HCl, and placed in a shaker for 5-10 hours. The above mixed solution was then placed in a dialysis bag (MWCO 7000) and dialyzed with PB overnight, during which water was replaced for five times. After loaded with different proportions (10%-30%) of drugs, the vesicles have a particle size of 112-121 nm, and a particle size distribution of 0.10-0.15. The encapsulation efficiency of DOX·HCl is 61%-77%. The in vitro release experiment was designed as above, and the release of drugs in the sample with 10 mM GSH was significantly faster than that in the sample without GSH.

The drug-carrying self-crosslinked polymeric vesicle GE11-CLPs based on PEG6.5k-P(CDC6k-co-TMC22.6k) was prepared by dialysis method, and doxorubicin hydrochloride (Epi·HCl) was loaded by pH gradient method. 80 µL of PEG5k-P(CDC5.8k-co-TMC23k) in DMF (10 mg/mL) and 20 µL of GE11-PEG6.5k-P(CDC6k-co-TMC22.6k) in DMF (10 mg /mL) were mixed uniformly, then directly placed into a dialysis bag (MWCO 7000), placed in a sodium citrate/citrate buffer solution (10 mM, pH 4.0), placed in a 37°C shaker for 4 hours to carry out self-crosslinked. After dialysis for the same medium for 12 hours, change the liquid five times. The cross-linked vesicles have a particle size of 96 nm with a particle size distribution of 0.18 measured by DLS. 0.05 mL of PB (4M, pH 8.5) was added to the above vesicle solution to establish a pH gradient, followed by immediately adding Epi·HCl and placing in a shaker for 5-10 hours. It was then placed in a dialysis bag (MWCO 7000) and dialyzed against PB overnight for five times. Loaded in different proportions (10%-30%), particle size 98-118 nm, particle size distribution 0.10-0.15, DOX·HCl package efficiency 64%-79%. The design of Epi·HCl in vitro release was the same as above, as shown in Figure 3. After adding 10 mM GSH, the drug was effectively released at a faster rate than the sample without GSH.

Similar preparation methods as described above can be used to study the drug loading capacity and encapsulation efficiency effects of various self-crosslinked polymeric vesicles and targeted self-crosslinked polymeric vesicles to a variety of hydrophilic anti-cancer small molecule drugs and genes, such as doxorubicin hydrochloride (DOX·HCl), Epirubicin hydrochloride (Epi·HCl), irinotecan hydrochloride (CPT·HCl) and mitoxantrone hydrochloride (MTO·HCl) and the hydrophobic anticancer drugs paclitaxel, docetaxel and olaparib. The loading amount and package efficiency are shown in Table 3.

**Table 3 Drug loading capacity for hydrophilic drugs and encapsulation efficiency of self-crosslinked polymeric vesicles and targeting self-crosslinked polymeric vesicles**

| Polymer/drug | Feed ratio (wt. %) | Drug loadi ng (wt. %) | encapsula tion rate (%) | Parti cle size (nm) | particle size distribut ion |
|---|---|---|---|---|---|
| PEG5k-P(CDC3.9k-*co*-LA14.6k)/DOX·HCl | 0 | -- | -- | 123 | 0.05 |
| | 10 | 6.5 | 69.7 | 134 | 0.14 |
| | 20 | 12.3 | 69.9 | 142 | 0.18 |
| | 30 | 20.9 | 88.3 | 153 | 0.18 |
| PEG5k-P(CDC5.8k-co-TMC23k)/DOX·HCl | 0 | -- | -- | 102 | 0.11 |
| | 10 | 7.1 | 76.5 | 105 | 0.11 |
| | 20 | 11.9 | 67.6 | 108 | 0.12 |
| | 30 | 15.9 | 62.9 | 124 | 0.15 |
| FA20/PEG5k-P(CDC5.8k-co-TMC23k)/DOX·HCl* | 0 | -- | -- | 88 | 0.08 |
| | 10 | 7.2 | 77.2 | 112 | 0.10 |
| | 20 | 11.8 | 66.8 | 116 | 0.13 |
| | 30 | 15.4 | 60.5 | 121 | 0.15 |
| GE1120/PEG5k-P(CDC5.8k-co-TMC23k)/DOX·HCl | 0 | -- | -- | 96 | 0.18 |
| | 10 | 7.3 | 79.1 | 98 | 0.10 |
| | 15 | 10.3 | 76.8 | 105 | 0.13 |
| | 20 | 11.4 | 64.3 | 118 | 0.15 |
| transferrin-PEG6.5k-P(CDC3.8k-*co*-LA13.8k)/DOX·H C1 | 0 | -- | -- | 134 | 0.09 |
| | 20 | 13.9 | 80.5 | 168 | 0.21 |
| PEG5k-P(CDC4.9k-TMC14.1k)/DOX·HCl | 0 | -- | -- | 40 | 0.12 |
| | 20 | 11.9 | 67.3 | 52 | 0.18 |
| PEG7.5k-P(CDC4.9k-TMC14.1k)/DOX·HCl | 0 | -- | -- | 57 | 0.19 |
| | 20 | 11.5 | 64.8 | 63 | 0.17 |
| P(CDC3.9k-TMC18.8k)-PEG5k-P(CDC3.9k-TMC18.8 k)/Epi·HCl* | 0 | -- | -- | 143 | 0.21 |
| | 20 | 12.6 | 71.8 | 172 | 0.26 |
| FA/PEG5k-P(CDC5.8k-co-TMC23k)/CPT·HCl* | 20 | 12.6 | 72.1 | 138 | 0.18 |
| GE11/PEG5k-P(CDC5.8k-co-TMC23k)/MTO·HCl | 20 | 6.7 | 35.8 | 108 | 0.10 |
| transferrin/P(CDC3.9k-LA18.8k)-PEG4k-P(CDC3.9k-LA18.8k)/CPT·HCl* | 20 | 11.9 | 67.5 | 121 | 0.06 |
| Ally-PEG6k-P(CDC4.9k-CL14.2k)/PTX | 20 | 13.5 | 78.1 | 135 | 0.17 |

### Starred examples are included for reference purposes.

### Example 21 Test of the toxicity of the empty self-crosslinked polymeric vesicles to SKOV3 Cells by MTT Method

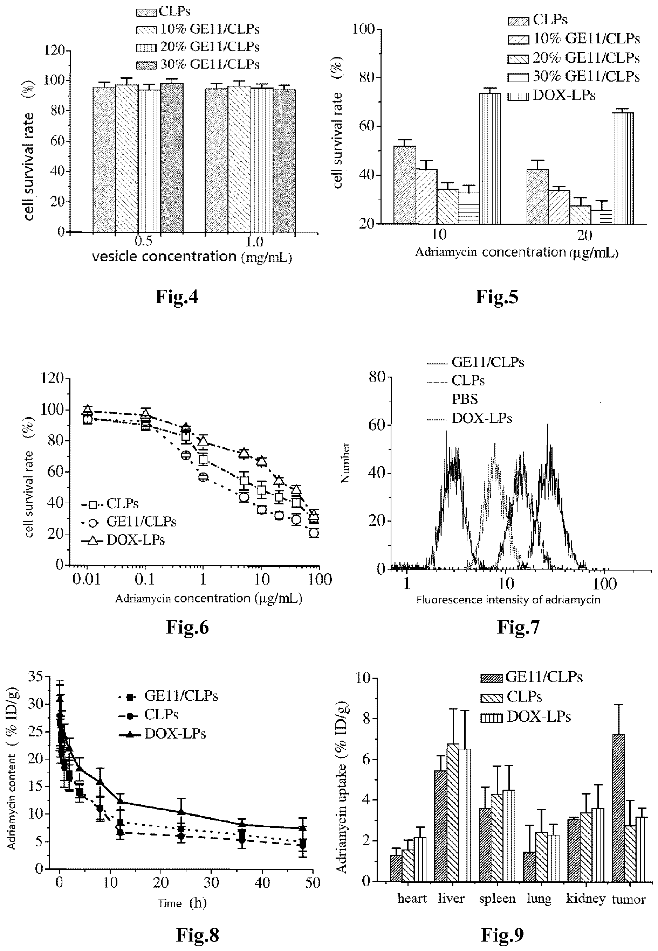

The cytotoxicity of the empty polymeric vesicles to SKOV3 human ovarian cancer cells was tested by MTT method. SKOV3 cells were seeded in 96-well plates at 5×10⁴ cells/mL, 100 µL per well, and the cells were cultured for 24 hours until 70% of cells were adhered to wall. Then, vesicle samples (such as non-loading self-crosslinked polymeric vesicles of Example 15 and Example 19) with different concentrations (0.5, 1.0 mg/mL) were added to each well of the experimental group. Cells of control well and medium of control wells (4 wells repeated) were further arranged. After 24 hours of culture, 10 µL of MTT (5.0 mg/mL) was added to each well for further incubation for 4 hours, then 150 µL of DMSO was added to each well to dissolve the crystals produced, and the absorbance value (A) was measured at 492 nm using a microplate reader to calculate cell viability with the medium of control well as zero. Figure 4 is a graph illustrating the cytotoxicity of self-crosslinked polymeric vesicles. It can be found that when the concentration of self-crosslinked polymeric vesicles increased from 0.5 to 1.0 mg/mL, the survival rate of SKOV3 was still higher than 92% indicating that the self-crosslinked polymeric vesicles of the present invention have good biocompatibility.

### Example 22 Test of Toxicity of drug-loaded self-crosslinked polymeric vesicles to SKOV3 ovarian cancer cells by MTT Method

The culture of cells was the same as that in Example 21 except that: DOX·HCl-loaded self-crosslinked polymeric vesicles PEG5k-P(CDC5.8k-co-TMC23k), DOX·HCl-loaded self-crosslinked polymeric vesicles GE11-CLPs composed of PEG5k-P(CDC5.8k-co-TMC23k) and GE11-PEG6.5k-P(CDC6k-co-TMC22.6k) (wherein GE11 were in an amount of 10%, 20%, 30% respectively) were added to each corresponding well of the experimental group. The concentration of DOX·HCl were 0.01, 0.1, 0.5, 1, 5, 10, 20, 40 and 80 µg/mL; and the targeting molecules were in an amount of 10%, 20% and 30%. The adriamycin liposomes Libod group was set as a control group. After 4 hours of co-cultivation, the samples were aspirated and cultured in fresh medium for a further cultivation for 44 h. The addition and process of MTT and the measuring of the absorbance were the same as in Example 21. Figure 5 and Figure 6 show the toxicity of drug-loaded self-crosslinked polymeric vesicles GE11-CLPsGE11 to SKOV3 cells. It can be found that 20% DOX·HCl-loaded self-crosslinked polymeric vesicles GE11-CLPsGE11 loading have a semi-lethal concentration (IC50) of 2.01 µg/mL to SKOV3 cells, which is much lower than that of self-crosslinked polymeric vesicles PEG5k-P(CDC5.8k-co-TMC23k), and also lower than that of adriamycin liposomes Libod (14.23 µg/mL), indicating that the drug-carrying self-crosslinked polymeric vesicles of the present invention can effectively target ovarian cancer cells to release drugs in cells, and finally kill cancer cells.

### Example 23 Test of toxicity of drug-loaded self-crosslinked polymeric vesicles to A2780 cells by MTT method

The culture of the cells was the same as in Example 21, except adding the self-crosslinked polymeric vesicles with different transferrin contents and different doses of drugs for the wells of the experimental group. For example, CPT·HCl, and self-crosslinked polymeric vesicles transferrin-CLPs prepared from Mal-PEG6k-P(CDC3.6k-LA18.6k) and P(CDC3.8k-LA18.8k)-PEG4k-P(CDC3.8k-LA18.8k) (Example 19) were added to corresponding wells, wherein the concentration of CPT·HCl is 0.01, 0.1, 0.5, 1, 5, 10, 20 and 40 µg/mL; the targeting molecular is in an amount of 10%, 20% and 30%; and the drug-carrying crosslinking polymeric vesicles P(CDC3.8k-LA18.8k)-PEG4k-P(CDC3.8k-LA18.8k) and a CPT·HCl group were set as a control group. After 4 hours of co-cultivation, the samples were aspirated and cultured in fresh medium for a further 44 h. Subsequent addition, process and measurement for absorbance of MTT were the same as in Example 21. The results showed that the IC₅₀ of the drug-loaded self-crosslinked polymeric vesicles P(CDC3.8k-LA18.8k)-PEG4k-P(CDC3.8k-LA18.8k) to A2780 cells was 4.15 µg/mL, and the IC₅₀ of 30% transferrin-CLPs carrying CPT·HCl to A2780 cells is 2.07 µg/mL, which was lower than that of the drug CPT·HCl (4.11 µg/mL).

Therefore, the drug-loaded self-crosslinked polymeric vesicle of the invention can effectively target ovarian cancer cells to release drugs in cells, and finally kill cancer cells. Especially the binding of targeting molecules greatly enhances the specificity to ovarian cancer cells and significantly increases the lethality of drugs against ovarian cancer cells.

The toxicity of various drug-loaded self-crosslinked polymeric vesicles on ovarian cancer cells was studied by similar methods described above. The drugs are a hydrophilic anti-cancer small molecule drug and the gene drug, such as doxorubicin hydrochloride (DOX·HCl), Epirubicin hydrochloride (Epi·HCl), irinotecan hydrochloride (CPT·HCl) and mitoxantrone hydrochloride (MTO·HCl) and the hydrophobic anticancer drugs, such as paclitaxel, docetaxel and olaparib, and the results are shown in Table 4.

### Example 24 Endocytosis of drug-loaded self-crosslinked polymeric vesicles (CLPs and GE11-CLPs)

Flow cytometry was used to test the endocytosis of drug-carrying vesicles using SKOV3 human ovarian cancer cells. SKOV3 cells were seeded in 6-well plates at 2×10⁵ cells/mL, 900 µL per well, and the cells were cultured for 24 hours until 70% cells are adherent to wall. Then, the drug-carrying vesicles samples CLPs and GE11-CLPs were added to each well of the experimental group. Cells of control well and medium of control wells (2 wells repeated) were further arranged. After 4 hours of culture, each well was trypsinized for 5 minutes, and washed three times with physiological saline. The fluorescence absorption intensity was measured at 488 nm by flow cytometry, and the endocytosis amount was calculated by using the saline group as a control. Figure 7 shows the results of cell uptake of drug-loaded cross-linked polymeric vesicles. It can be found that the uptake of cells by targeted drug-loaded self-crosslinked polymeric vesicles is higher than that of non-targeting drug-carrying self-crosslinked polymeric vesicles and adriamycin liposomes Libod, indicating that the targeted self-crosslinked polymeric vesicles can be actively taken up via endocytosis by ovarian cancer cells.

### Reference Example 25 Blood circulation of drug-loaded self-crosslinked polymeric vesicles (CLPs and FA-CLPs)

All animal experiments were conducted in accordance with the regulations of the Animal Experimental Center of Suzhou University. Balb/C nude mice of 18-20 grams and 4-6 weeks old were used in the experiments. The vesicles were prepared by mixing FA-PEG6.5k-P(CDC6k-co-TMC22.6k) and PEG5k-P(CDC5.8k-co-TMC23k) in different proportions, designated as FA-CLPs. When the proportion of FA in the polymeric vesicles was 20%, the particle size is 100 nm, the particle size distribution is 0.10, and the polymeric vesicles was designated as FA20-CLPs, the drug was DOX·HCl DOX·HCl-loaded CLPs vesicles, FA-CLPs vesicles, and DOX·HCl were injected into mice via tail vein (DOX dose of 10 mg/kg). 10 µL blood was taken after 0, 0.25, 0.5, 1, 2, 4, 8, 12 and 24 hours respectively. The blood was accurately weighed by the differential method, then 100 µL of 1% Triton and 500 µL of extraction liquid (DMF comprising 20 mM DTT and 1 M HCl) were added; then centrifugation (20,000 rpm, 20 minutes) was performed to obtain a supernatant. The amount of DOX·HCl at each time point was measured by fluorescence. It can be found from the calculation that the elimination half-lives of drug-loaded self-crosslinked polymeric vesicles FA-CLPs and drug- loaded self-crosslinked polymeric vesicles CLPs in mice were 4.23 and 4.16 hours respectively, while DOX·HCl has an elimination half-life of 0.27 hours. Therefore the self-crosslinked polymeric vesicles disclosed herein are stable in mice and have a long cycle time. The operation and calculation methods of the blood circulation experiments for other drug-carrying self-crosslinked polymeric vesicles were the same with the above description, and the results are shown in Table 4.

### Example 26 Blood circulation of drug- loaded self-crosslinked polymeric vesicle CLPs and GE11-CLPs

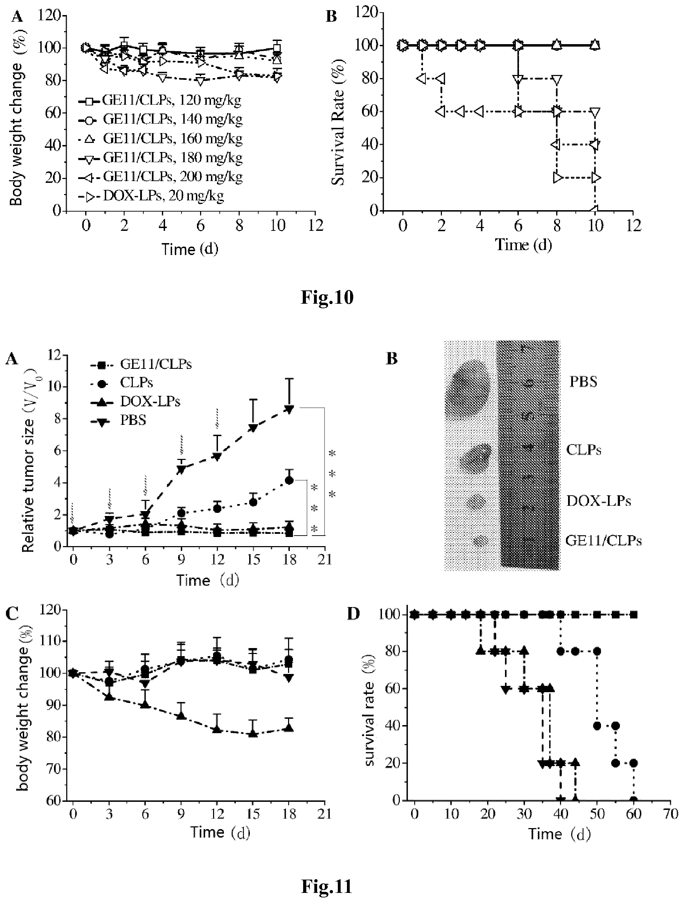

As in Reference Example 25, self-crosslinked polymeric vesicles GE11-CLPs were prepared by mixing GE11-PEG6.5k-P(CDC6k-co-TMC22.6k) and PEG5k-P(CDC5.8k-co-TMC23k). Self-crosslinked polymeric vesicles GE11-CLPs and self-crosslinked polymeric vesicles CLPs were loaded with DOX·HCl, and then injected into Balb/C nude mice via tail vein to study blood circulation thereof, wherein DOX·HCl and Libod DOX-LPs were used for control group. As shown in Fig. 10, the GE11-CLPs and CLPs vesicles remained 5.0 ID%/g after 48 hours. It can be calculated that the elimination half-life of self-crosslinked polymeric vesicles GE11-CLPs and self-crosslinked polymeric vesicles CLPs in mice are 4.99 and 4.79 hours respectively, therefore they are stable in mice and have a long cycle time. The results are shown in Table 4.

### Example 27 In vivo imaging of self-crosslinked polymeric vesicles FA-CLPs in SKOV3 ovarian cancer mice

In vivo imaging experiments were performed on Balb/C nude mice of 4-6 weeks old weighing about 18-20 g. The mice were subcutaneously injected with 5×10⁶ SKOV3 human ovarian cancer cells. The experiments were started after about 3∼4 weeks when the tumor grows to 100∼200 mm³. Self-crosslinked polymeric vesicles FA20-CLPs prepared by mixing FA-PEG6.5k-P(CDC6k-co-TMC22.6k) with PEG5k-P(CDC5.8k-co-TMC23k) in a ratio of 1:5 and self-crosslinked polymeric vesicles CLPs are exemplified. The FA20-CLPs labeled by fluorescent substance cy-7 and the non-targeted CLPs were injected into the mice via tail vein, and then the vesicles were tracked at different time points 1, 2, 4, 6, 8, 12, 24, 48 hours with living small animal optical imaging system. The experimental results show that FA20-CLPs accumulate rapidly at the tumor site, and the fluorescence is still strong after 48 hours. These results indicate that FA20-CLPs can actively target ovarian cancer tumor sites and be enriched there, showing strong specificity for ovarian cancer cells. The operation and calculation methods of the in vivo imaging experiments for other self-crosslinked polymeric vesicles were the same with the above description, and the results are shown in Table 4.

Epi·HCl-loaded cy-7-labeled CLPs and GE11-CLPs were prepared to perform the *in vivo* imaging experiments, wherein the tumor inoculation and tail vein administration were the same as the above description. The both vesicles were found to accumulate rapidly at ovarian tumor sites. LPs disappeared in 4-6 hour, but the fluorescence of the tumor site with GE11-CLPs remained strong after 48 hours, indicating that GE11-CLPs can actively target the ovarian tumor site and be enriched there.

### Example 28 In vivo imaging experiments of drug- loaded self-crosslinked polymeric vesicle CLPs and transferrin-CLPs in A2780 ovarian cancer mice

In vivo imaging experiments were performed on Balb/C nude mice of 4-6 weeks old weighing about 18-20 g. The mice were subcutaneously injected 5×10⁶ A2780 human ovarian cancer cells. The experiments were started after about 3∼4 weeks when the tumor grows to 100-200 mm³.

Targeting self-crosslinked polymeric vesicles transferrin-CLPs prepared by mixing ferferrin-PEG6.5k-P(CDC3.8k-co-LA13.8k) and PEG5k-P(CDC3.7k-co-LA14.6k) and drug-loaded self-crosslinked polymeric vesicles CLPs were labeled with cy-5 and loaded with the hydrophobic drug docetaxel DTX. The same procedure as in Example 27 was used to study in vivo imaging. The experimental results show that the DTX carrying-transferrin-CLPs can accumulate rapidly in the tumor site, and the fluorescence of that tumor site was still strong after 48 hours indicating that transferrin-CLPs can actively target tumor sites and be enriched there, while drug- loaded CLPs self-crosslinked polymeric vesicles metabolize quickly after entering the tumor in 2 hours, and the fluorescence intensity is low.

### Reference Example 29 Biodistribution of drug- loaded self-crosslinked polymeric vesicles CLPs and FA-CLPs in SKOV3 ovarian cancer mice

Preparation of sample FA20-CLPs and inoculation of tumors and tail vein administration in biodistribution experiments were the same as in Example 27. FA20-CLPs and CLPs were injected into mice (DOX·HCl, 10 mg/kg). The mice were sacrificed after 12 hours, and the tumor, heart, liver, spleen, lung and kidney tissues were obtained and washed and weighed, then 500 µL 1% Triton was added respectively, and the tissue was grounded by a homogenizer and then extracted with 900 µL of DMF (comprising 20 mM DTT, 1 M HCl). After centrifugation (20,000 rpm for 20 minutes), a supernatant was obtained and measured by fluorescence at each time point for the amount of DOX·HCl. In Figure 8, the horizontal axis represents the tissue organ, and the vertical axis represents the amount of DOX·HCl account for total DOX·HCl injection (ID%/g) per gram of tumor or tissue. The amount of DOX·HCl accumulated in tumors 12 hours after injection of FA-CLPs, CLPs and DOX·HCl were 6.54, 2.53 and 1.02 ID%/g respectively. The amount of DOX·HCl for FA-CLPs were 3 and 6 times higher than that of CLPs and DOX·HCl, indicating drug-loaded FA-CLPs accumulate more at the tumor site by active targeting, and have significant specificity to ovarian cancer cells, which is beneficial for killing ovarian cancer cells. The results are shown in Table 4.

### Example 30 Biodistribution of drug- loaded self-crosslinked polymeric vesicle CLPs and GE11-CLPs in SKOV3 ovarian cancer mice

Tumor inoculation, tail vein administration, and operations for animals are the same as in Example 27. GE11-CLPs and CLPs carrying DOX·HCl and liposomal doxorubicin Libod DOX-LPs were injected into mice (DOX·HCl: 10 mg/kg) via tail vein. After 6 hours, the amount of DOX·HCl accumulated in the tumors for GE11-CLPs, CLPs and DOX-LP were 8.63, 3.52 and 1.82 ID%/g, respectively, and the amount of DOX·HCl for GE11-CLPs were 2 and 5 times higher than the latter two, indicating that the drug- loaded GE11CLP accumulates more at the tumor site by active targeting (Fig. 9).

### Example 31 The maximum tolerated dose (MTD) of Balb/C mice to drug- loaded self-crosslinked polymeric vesicles GE11-CLPs

Balb/C nude mice of 4∼6 weeks old, weighing about 18∼20 g were used. Self-crosslinked polymeric vesicles GE11-CLPs and doxorubicin liposomes Libod (including doxorubicin at concentrations of 120 mg/kg, 140 mg/kg, 160 mg/kg, 180 mg/kg and 200 mg/kg,) were injected at a single-dose, and the concentration of doxorubicin liposomes Libod was 20 mg/kg. There are five mice in each group. The mental state of the mice was observed and the body weight was measured for the last 10 days before executed. The standard of the maximum tolerated dose was that the mice did not die accidentally or the body weight of the mice did not decrease to below 15% of the mice. It can be seen from the changes of body weight and survival rate of the mice for each component in Figures 10A and 10B, the maximum tolerated dose of the drug- loaded targeted self-crosslinked polymeric vesicles was 160 mg/kg, while the maximum tolerated dose of doxorubicin liposomes Libod was 20 mg/kg. Therefore, the mice have high tolerance to targeted drug- loaded self-crosslinked polymeric vesicles which greatly improves the therapeutic window.

### Example 32 Antitumor effect of drug- loaded self-crosslinked polymeric vesicles GE11-CLPs and CLPs and effects thereof to body weight change and survival rate for mice bearing SKOV3 subcutaneous ovarian cancer

Balb/C nude mice of 4-6 weeks old, weighing about 18-20 g were injected subcutaneously with 5×10⁶ SKOV3 human ovarian cancer cells. The experiment was started after about two weeks when the tumor grows to 30∼50 mm³. DOX·HCl- loaded self-crosslinked polymeric vesicles GE11-CLPs prepared by mixing GE11-PEG6.5k-P(CDC6k-co-TMC22.6k) and PEG5k-P(CDC5.8k-co-TMC23k) at 1:5, the non-targeted CLPs, DOX-LPs, and PBS were injected via tail vein. As shown in Fig. 11, tumors were significantly inhibited in the GE11-CLPs treatment group at 18th day, while tumors in the drug-carrying CLPs group increased, and the body weight of the mice hardly changed. Although DOX-LPs also inhibited tumor growth, the body weight of mice in the DOX-LPs group decreased by 18% at 12th day, indicating serious toxic side effects thereof on mice. The GE11-CLPs treatment group survived after 62 days, the DOX-LPs group had all died at 42th day, and the PBS group also all died at 42th day. Therefore, drug- loaded self-crosslinked polymeric vesicles can effectively inhibit tumors, have no toxic side effects on mice, and prolong the survival time of tumor-bearing mice.

### Example 33 Antitumor effect of drug-carrying self-crosslinked polymeric vesicles GE11-CLPs at single dose and effects thereof to body weight change and survival rate for mice bearing SKOV3 subcutaneous ovarian cancer

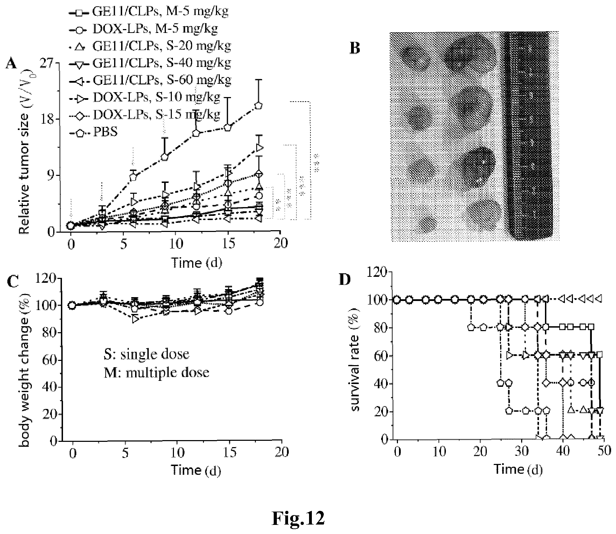

The establishment of subcutaneous SKOV3 tumor model, tail vein administration and data collection were the same as in Example 32. DOX·HCl -loaded GE11-CLPs, doxorubicin liposomes Libod DOX-LPs, and PBS were injected via tail vein at a single dose, wherein the DOX·HCl-loadedself-crosslinked polymeric vesicles GE11-CLPs had doxorubicin concentrations of 20 mg/kg, 40 mg/kg and 60 mg/kg, while the doxorubicin dose of DOX-LPs had doxorubicin concentrations of 10 mg/kg and 15 mg/kg. As shown in Fig. 12, in GE11-CLPs group with 60 mg/kg of doxorubicin, the tumors were significantly inhibited at 18th day, while in GE11-CLPs groups with 20 mg/kg and 40 mg/kg of doxorubicin, the tumors grew, and the body weight of the mice in all groups hardly changed. DOX-LPs with 10 mg/kg and 15 mg/kg of doxorubicin did not inhibit tumor growth at a single dose. The GE11-CLPs treatment group survived after 49th day, the DOX-LPs group had all died at 42th and 43th day, and the PBS group had also all died at 34th day.

### Example 34 Antitumor effect of drug-loading targeted self-crosslinked polymeric vesicles transferrin-CLPs and CLPs and effects thereof to body weight change and survival rate for mice bearing A2780 subcutaneous ovarian cancer

The establishment of the subcutaneous A2780 tumor model, way of tail vein administration and data collection were the same as in Example 32. The experiment was started at a tumor size of 30-50 mm³. CPT·HCl-loaded targeted self-crosslinked polymeric vesicles transferrin-CLPs prepared by mixing transferrin-PEG6.5k-P(CDC3.8k-co-LA13.8k) and PEG5k-P(CDC3.7k-co-LA14.6k) at 1:5, the non-targeted CLPs, free CPT·HCl were injected via tail vein. DOX·HCl-carrying self-crosslinked polymeric vesicle cRGD-CLPs prepared by mixing cRGD-PEG6k-P(CDC4.6k-co-TMC18.6k) and PEG5k-P(CDC4.9k-co-TMC19k) at 1:5 was set as a control group. The results showed that tumors were significantly inhibited at 18th day when treated with transferrin-CLPs, while tumors in the drug-carrying CLPs group showed a small increase, and the body weight of the mice didn't change. The weight of mice in the cRGD-CLPs group did not change, but the tumor inhibition was significantly weaker than the former, and the tumor size was 3 times that of the former, indicating that cRGD had no obvious targeting property to ovarian cancer. Although CPT·HCl also inhibited growth of tumor, the weight of mice in the CPT·HCl group decreased by 18% at 10th day. Mice in the transferrin-CLPs treatment group had all survived after 72 days, while mice in the CPT·HCl group had all died at 28th day, and the PBS group also had all died at 37th day.

### Example 35 Antitumor effect of drug-carrying targeting self-crosslinked polymeric vesicles GE11-CLPs and CLPs and effects thereof to body weight change and survival rate for mice bearing SKOV3 orthotopic ovarian cancer

DOX·HCl-carrying self-crosslinked polymeric vesicles GE11-CLPs, the non-targeted CLPs, DOX-LPs, and PBS were injected into mice bearing SKOV3 orthotopic ovarian cancer via tail vein. In the GE11-CLPs treatment group, the bioluminescence intensity of the tumor continued to decrease within 16 days, while the bioluminescence intensity of the tumor in drug-loaded CLP group increased to some extent, and the body weight of the mice hardly changed. Although DOX-LPs can also inhibit tumor growth, the body weight of mice in DOX-LPs group decreased by 21% at 4th day. The mice in GE11-CLPs treatment group had all survived after 45 days, the mice in DOX-LPs group had all died at 32th day, and the mice in PBS group had also all died at 23th day. Therefore, the drug-loaded self-crosslinked polymeric vesicles GE11-CLPs bonded to targeted molecules can effectively inhibit the growth of orthotopic ovarian cancer, have no toxic side effects, and can prolong the survival time of tumor-bearing mice.

The effects of various self-crosslinked polymeric vesicles loaded with different drugs on ovarian cancer-bearing mice were studied by the similar experimental methods described above. The results are shown in Table 4.

**Table 4 Antitumor results in vitro or in vivo of the drug-loaded self-crosslinked polymeric vesicles against ovarian cancer**

| Polymeric vesicles | 24 h /pH 7.4 release in vitro (%) | | Ovarian cancer cell survival rate % | | IC₅₀ (µg/mL) | Circulatory elimination half-life (h) | Drug accumulation in tumor (%ID/g) | mum tolerated amount (mg/k g) | Maxi survival time(day) | |
|---|---|---|---|---|---|---|---|---|---|---|
| | No GSH | 10 mM GSH | empty vesicles | drug carrying vesicles | | | | | subcutaneous mode 1 | orthotopic mode 1 |
| PEG5k-P(CDC5.8k-co-TMC23k)/ DOX·HCl | 14 | 78 | >90 | 43.5 | 8.92 | 4.79 | 3.52 | 120 | 38 | 40 |
| FA20-CLPs/PEG5k-P(CDC5.8k-co-TMC23k)/DOX ·HCl* | 21 | 81 | >90 | 21.6 | 2.13 | 4.49 | 6.54 | 160 | >60 | >45 |
| GE11-CLPs/PEG5k-P(CDC5.8k-co-TMC23k)/DOX·HCl | 23 | 82 | >91 | 16.8 | 1.92 | 4.99 | 8.63 | 160 | >62 | >45 |
| Transferrin-CLP s/PEG6.5k-P(CDC3.8k-*co*-LA13. 8k)/DTX | 24 | 76 | >88 | 22.7 | 3.06 | 5.04 | 9.02 | 150 | >72 | >40 |

Starred examples are included for reference purposes.

## Claims

1. An ovarian cancer specifically targeted biodegradable amphiphilic polymer, **characterized in that** the ovarian cancer specifically targeted biodegradable amphiphilic polymer is prepared by bonding a polymer containing dithiocarbonate monomer with a targeting molecule by Michael addition or amidation reaction;
wherein the targeting molecule is GE11 polypeptide or transferrin; and
wherein the polymer containing dithiocarbonate monomer has a chemical structure of Formula I: 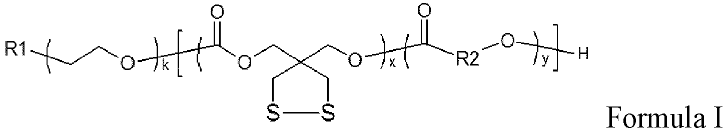 wherein R1 is selected from one of the following groups: 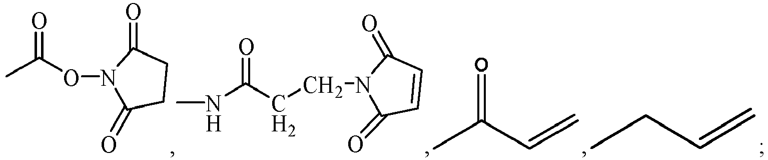 R2 is selected from one of the following groups: 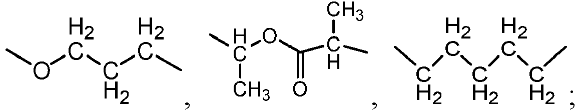 and
wherein k is from 113 to 170, x is from 15 to 45, and y is from 80 to 300, and
the polymer containing dithiocarbonate monomer has a molecular weight of 30 kDa to 55 kDa.

2. A polymeric vesicle, **characterized in that** the polymeric vesicle is prepared by one of the following preparation methods:
(1) by self-crosslinking of the ovarian cancer specifically targeted biodegradable amphiphilic polymer according to claim 1;
(2) by coupling a targeting molecule to the surface of the polymeric vesicles prepared by self-crosslinking of the polymer containing dithiocarbonate monomer as mentioned in claim 1, wherein the targeting molecule is GE11 polypeptide or transferrin;
and calculated according to percentage of mass, the ovarian cancer specifically targeted biodegradable amphiphilic polymer is in an amount of 1 wt.% to 40 wt.% by mass;
wherein the polymeric vesicle is a self-crosslinked polymeric vesicle; and has a particle size of 50 nm to 160 nm.

3. The polymeric vesicle according to claim 2 for use as a carrier of a drug for treating ovarian cancer.

4. The polymeric vesicle for use according to claim 3, **characterized in that** the drug for treating ovarian cancer is a small molecular anticancer drug.

5. The polymeric vesicle for use according to claim 4, wherein the small molecular anticancer drug is paclitaxel, docetaxel, adriamycin, olaparib, gefitinib, doxorubicin hydrochloride, epirubicin hydrochloride, or irinotecan hydrochloride.

6. The ovarian cancer specifically targeted biodegradable amphiphilic polymer according to claim 1 for use as a nanomedicine for treating ovarian cancer.

7. The polymeric vesicle according to claim 2 for use as a nanomedicine for treating ovarian cancer.

## Patentansprüche

1. Biologisch abbaubares, amphiphiles Polymer, das spezifisch auf einen Eierstockkrebs abzielt, **dadurch gekennzeichnet, dass** das biologisch abbaubare, amphiphiles Polymer, das spezifisch auf einen Eierstockkrebs abzielt, durch ein Binden eines Polymers, das ein Dithiocarbonatmonomer enthält, mit einem abzielenden Molekül durch eine Michael-Addition oder eine Amidierungsreaktion hergestellt wird;
wobei das abzielende Molekül GE11-Polypeptid oder Transferrin ist; und
wobei das Polymer, das ein Dithiocarbonatmonomer enthält, eine chemische Struktur der Formel I aufweist: 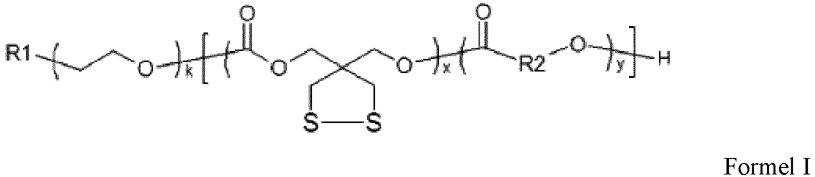 wobei R1 aus einer der folgenden Gruppen ausgewählt ist: 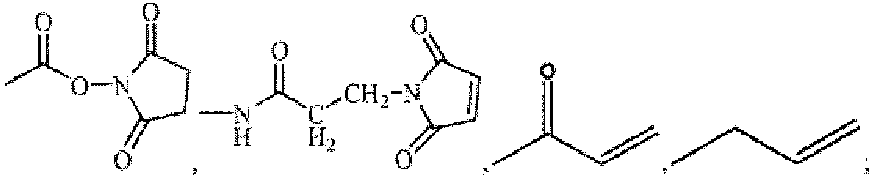 wobei R2 aus einer der folgenden Gruppen ausgewählt ist: 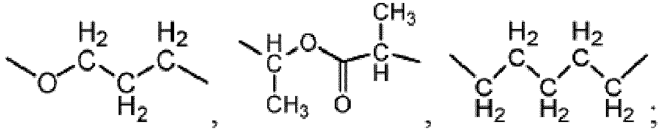 und
wobei k von 113 bis 170 ist, x von 15 bis 45 ist und y von 80 bis 300 ist und
das Polymer, das ein Dithiocarbonatmonomer enthält, ein Molekulargewicht von 30 kDA bis 55 kDA aufweist.

2. Polymervesikel, **dadurch gekennzeichnet, dass** die Polymervesikel durch eines der folgenden Herstellungsverfahren hergestellt wird:
(1) durch Selbstvernetzen des biologisch abbaubaren amphiphilen Polymer, das spezifisch auf Eierstockkrebs abzielt, nach Anspruch 1;
(2) durch Koppeln eines abzielenden Moleküls an der Oberfläche der Polymervesikel, die durch das Selbstvernetzen des Polymers, das ein Dithiocarbonatmonomer enthält, nach Anspruch 1 hergestellt wird, wobei das abzielende Molekül GE11-Polypeptid oder Transferrin ist;
und berechnet gemäß eines Masseprozentsatzes das biologisch abbaubare amphiphile Polymer, das spezifisch auf Eierstockkrebs abzielt, in einer Menge von 1 Massengew.-% bis 40 Massengew.-% vorliegt;
wobei die Polymervesikel eine selbstvernetzte Polymervesikel ist; und
eine Partikelgröße von 50 nm bis 160 nm aufweist.

3. Polymervesikel nach Anspruch 2 für die Verwendung als ein Träger eines Arzneimittels für das Behandeln von Eierstockkrebs.

4. Polymervesikel für die Verwendung nach Anspruch 3, **dadurch gekennzeichnet, dass** das Arzneimittel für das Behandeln von Eierstockkrebs ein kleinmolekulares Antikrebsarzneimittel ist.

5. Polymervesikel für die Verwendung nach Anspruch 4, wobei das kleinmolekulare Antikrebsarzneimittel Paclitaxel, Docetaxel, Adriamycin, Olaparib, Gefitinib, Doxorubicinhydrochlorid, Epirubicinhydrochlorid oder Irinotecanhydrochlorid ist.

6. Biologisch abbaubares, amphiphiles Polymer, das spezifisch auf einen Eierstockkrebs abzielt, nach Anspruch 1 für die Verwendung als eine Nanomedizin für das Behandeln von Eierstockkrebs.

7. Polymervesikel nach Anspruch 2 für die Verwendung als eine Nanomedizin für das Behandeln von Eierstockkrebs.

## Revendications

1. Polymère amphiphile biodégradable spécifiquement ciblé contre le cancer de l'ovaire, **caractérisé en ce que** le polymère amphiphile biodégradable spécifiquement ciblé contre le cancer de l'ovaire est préparé par liaison d'un polymère contenant un monomère de dithiocarbonate avec une molécule de ciblage par addition de Michael ou réaction d'amidation ;
dans lequel la molécule de ciblage est le polypeptide GE11 ou la transferrine ; et
dans lequel le polymère contenant un monomère de dithiocarbonate a une structure chimique de formule I : 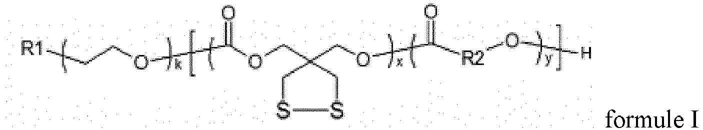 dans lequel R1 est choisi parmi l'un des groupes suivants : 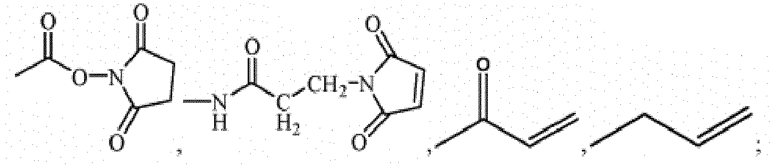 R2 est choisi parmi l'un des groupes suivants : 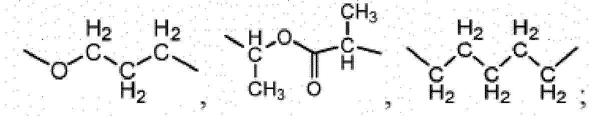 et
dans lequel k est de 113 à 170, x est de 15 à 45, et y est de 80 à 300, et
le polymère contenant un monomère de dithiocarbonate a un poids moléculaire de 30 kDa à 55 kDa.

2. Vésicule polymère, **caractérisée en ce que** la vésicule polymère est préparée par l'un des procédés de préparation suivants :
(1) par auto-réticulation du polymère amphiphile biodégradable spécifiquement ciblé contre le cancer de l'ovaire selon la revendication 1 ;
(2) en couplant une molécule de ciblage à la surface des vésicules polymères préparées par auto-réticulation du polymère contenant un monomère de dithiocarbonate tel que mentionné dans la revendication 1, dans laquelle la molécule de ciblage est le polypeptide GE11 ou la transferrine; et calculé en fonction du pourcentage de masse, le polymère amphiphile biodégradable spécifiquement ciblé contre le cancer de l'ovaire est en une quantité de 1% en poids à 40% en poids ;
dans laquelle la vésicule polymère est une vésicule polymère auto-réticulée ; et a une taille de particule de 50 nm à 160 nm.

3. Vésicule polymère selon la revendication 2, destinée à utiliser comme vecteur d'un médicament pour traiter le cancer de l'ovaire.

4. Vésicule polymère à utiliser selon la revendication 3, **caractérisée en ce que** le médicament pour traiter le cancer de l'ovaire est un médicament anticancéreux de petite taille moléculaire.

5. Vésicule polymère à utiliser selon la revendication 4, dans laquelle le médicament anticancéreux de petite taille moléculaire est le paclitaxel, le docétaxel, l'adriamycine, l'olaparib, le géfitinib, le chlorhydrate de doxorubicine, le chlorhydrate d'épirubicine ou le chlorhydrate d'irinotécan.

6. Polymère amphiphile biodégradable spécifiquement ciblé contre le cancer de l'ovaire selon la revendication 1 à utiliser comme nanomédecine pour traiter le cancer de l'ovaire.

7. Vésicule polymère selon la revendication 2 à utiliser comme nanomédecine pour traiter le cancer de l'ovaire.
